(19) Europäisches Patentamt
European Patent Office
Office européen des brevets

(11) EP 2 746 270 A1

(12) EUROPEAN PATENT APPLICATION
published in accordance with Art. 153(4) EPC

(43) Date of publication:
**25.06.2014 Bulletin 2014/26**

(21) Application number: **12832975.2**

(22) Date of filing: **14.09.2012**

(51) Int Cl.:
***C07D 323/00*** (2006.01) ***C08G 65/40*** (2006.01)

(86) International application number:
**PCT/JP2012/005896**

(87) International publication number:
**WO 2013/042343 (28.03.2013 Gazette 2013/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.09.2011 JP 2011205923**
**21.09.2011 JP 2011205922**
**23.02.2012 JP 2012037529**
**31.07.2012 JP 2012169396**

(71) Applicant: **Toray Industries, Inc.**
**Tokyo, 103-8666 (JP)**

(72) Inventors:
- **YAMASHITA, Kohei**
**Nagoya-shi**
**Aichi 455-8502 (JP)**
- **YOKOE, Makito**
**Nagoya-shi**
**Aichi 455-8502 (JP)**
- **HORIUCHI, Shunsuke**
**Nagoya-shi**
**Aichi 455-8502 (JP)**
- **YAMAUCHI, Koji**
**Nagoya-shi**
**Aichi 455-8502 (JP)**

(74) Representative: **Webster, Jeremy Mark et al**
**Mewburn Ellis LLP**
**33 Gutter Lane**
**London**
**EC2V 8AS (GB)**

# (54) METHOD FOR RECOVERING CYCLIC POLYPHENYLENE ETHER ETHER KETONE COMPOSITION AND METHOD FOR PRODUCING POLYPHENYLENE ETHER ETHER KETONE USING SAME

(57) The object of the invention is to provide a method of economically and efficiently recovering an industrially useful cyclic poly(phenylene ether ether ketone) composition in a short time. A mixture including linear poly(phenylene ether ether ketone) and cyclic poly(phenylene ether ether ketone) is exposed to a ketone-based solvent and is then subjected to solid-liquid separation. This enables recovery of a cyclic poly(phenylene ether ether ketone) composition including a cyclic poly(phenylene ether ether ketone) mixture of cyclic poly(phenylene ether ether ketone)s having different repeating numbers m.

EP 2 746 270 A1

## Description

### Technical Field

The present invention relates to a recovery method of a cyclic poly(phenylene ether ether ketone) composition and a production method of poly(phenylene ether ether ketone) using the same.

### Background Art

Poly(phenylene ether ether ketone) is a thermoplastic resin having extremely high heat resistance. The poly(phenylene ether ether ketone) is also one type of super engineering plastics having excellent chemical resistance and flame retardancy and high mechanical strength and dimensional stability. The poly(phenylene ether ether ketone) is used in applications of automobile components by taking advantage of its characteristics. Specifically, the poly(phenylene ether ether ketone) is used as a metal substitution material, with a view to improving the performance of engine components and reducing the weight of engine components.

Cyclic poly(phenylene ether ether ketone) is a cyclic compound of poly(phenylene ether ether ketone). The cyclic poly(phenylene ether ether ketone) has the cyclic structure-induced characteristics as an inclusion compound, like other aromatic cyclic compounds. The cyclic poly(phenylene ether ether ketone) is also used as a monomer for synthesis of a high molecular-weight linear polymer by ring-opening polymerization.

The following method has been proposed as a general production method of poly(phenylene ether ether ketone). A dihalogenated aromatic ketone compound such as 4,4'-difluorobenzophenone and hydroquinone are made to react in a solvent of diphenylsulfone under a basic condition. This reaction provides a reaction mixture including poly(phenylene ether ether ketone). Diphenylsulfone is then extracted out from the reaction mixture by using acetone, methanol or ethanol, so that poly(phenylene ether ether ketone) is recovered (see, for example, Patent Documents 1 and 2). This method is widely used as an industrial production method of poly(phenylene ether ether ketone). This method enables recovery of poly(phenylene ether ether ketone) of high purity, but does not refer at all to recovery of cyclic poly(phenylene ether ether ketone).

A production method using sulfolane as a polymerization solvent has also been proposed as another production method of poly(phenylene ether ether ketone) (see, for example, Patent Document 3). Sulfolane is water soluble, unlike diphenylsulfone generally used for industrial manufacture of poly(phenylene ether ether ketone). This method accordingly has an advantage that poly(phenylene ether ether ketone) can be recovered from a reaction mixture only by washing with water. Sulfolane has, however, low thermal stability in a high temperature region and is thus difficult to be handled. Additionally, this method also does not refer at all to recovery of cyclic poly(phenylene ether ether ketone).

The following method has been disclosed, on the other hand, as a recovery method of cyclic poly(phenylene ether ether ketone) (see, for example, Non-Patent Document 1). The method first extracts a mixture including linear poly(phenylene ether ether ketone) and cyclic poly(phenylene ether ether ketone) with chloroform. The method then purifies a chloroform-soluble component by silica gel chromatography using methylene chloride and thereby recovers cyclic poly(phenylene ether ether ketone) of high purity. This method enables recovery of cyclic poly(phenylene ether ether ketone) of high purity. This method is, however, a recovery method using large amounts of chlorine-based solvents having significant environmental burden, such as chloroform and methylene chloride as necessary requirements. This method accordingly has little industrial availability. According to Non-Patent Document 1, the mixture including the linear poly(phenylene ether ether ketone) and the cyclic poly(phenylene ether ether ketone) is obtained by reaction of a linear poly(phenylene ether ether ketone) oligomer having hydroxyl groups at both ends with a linear poly(phenylene ether ether ketone) oligomer having fluoride groups at both ends as shown by a formula below.

[Chem. 1]

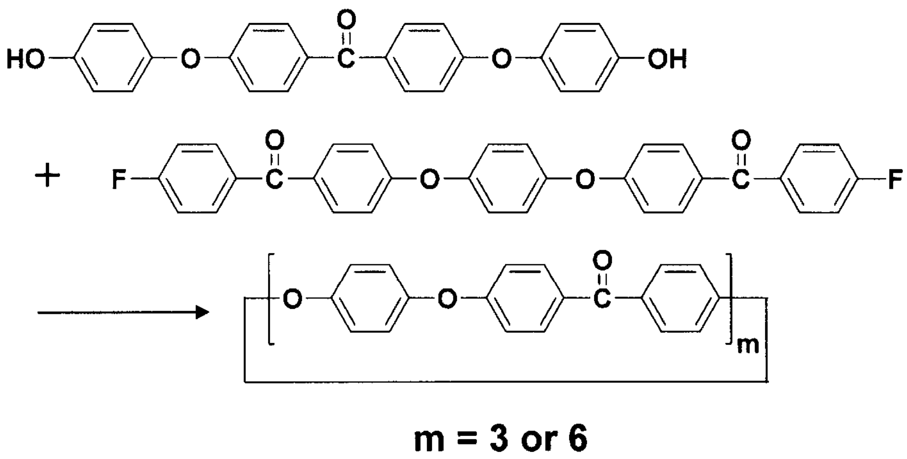

A cyclic poly(phenylene ether ether ketone) mixture obtained by this method includes cyclic poly(phenylene ether ether ketone)s having repeating numbers m= 3 and/or 6. Accordingly, this method provides only a cyclic poly(phenylene ether ether ketone) having a melting point of higher than 270°C. The cyclic poly(phenylene ether ether ketone) obtained from the linear oligomers shown by the above formula (oligomer having hydroxyl groups at both ends and consisting of four units of benzene ring component and oligomer having fluoride groups at both ends and consisting of five units of benzene ring component) consists of only a cyclic trimer (m= 3) and a cyclic hexamer (m= 6). It is described that these are cyclic poly(phenylene ether ether ketone)s respectively have a melting point of 366°C and a melting point of 324°C.

As a recovery method of a cyclic poly(phenylene ether ether ketone) composition from a mixture including linear poly(phenylene ether ether ketone) and cyclic poly(phenylene ether ether ketone), recovery from a solvent extraction-soluble component has been disclosed, wherein chloroform is specified as a preferable extraction solvent (see, for example, Patent Document 4). Using chloroform as the extraction solvent enables recovery of cyclic poly(phenylene ether ether ketone) of high purity. This method, however, needs a large amount of chloroform which is a chlorine-based solvent having significant environmental burden. A chloroform-substituting extraction solvent has accordingly been demanded.

Non-Patent Document 2 discloses a method shown by a formula given below as a recovery method of cyclic poly(phenylene ether ether ketone) without using a chlorine-based solvent such as chloroform. Linear poly(phenylene ether ether ketone) having hydroxyl groups at both ends is made to react with 4,4'-difluorobenzophenone under a pseudo high dilution condition. A mixture of linear poly(phenylene ether ether ketone) and cyclic poly(phenylene ether ether ketone) obtained by the reaction is recovered by acetone extraction and recrystallization purification using dimethylsulfoxide (DMSO). The "reaction under the pseudo high dilution condition" means a technique that makes the concentration of a reactant in a reaction system to be in a pseudo-diluted state by gradually adding the reactant in the reaction system during the reaction.

[Chem. 2]

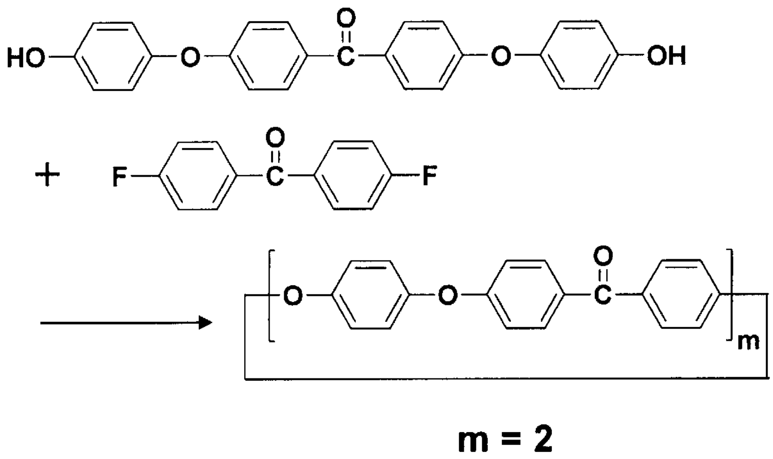

This method also enables recovery of cyclic poly(phenylene ether ether ketone) of high purity. This method seems, however, not to be an industrially available recovery method, due to the need of using a large amount of DMSO as the recrystallization solvent relative to cyclic poly(phenylene ether ether ketone) and the potential loss of a bulk of cyclic poly(phenylene ether ether ketone) by recrystallization. Additionally, linear poly(phenylene ether ether ketone) is used as a synthetic raw material, so that the cyclic poly(phenylene ether ether ketone) obtained by this method is limited to a cyclic dimer (m= 2) having an extremely high melting point.

Non-Patent Document 3 discloses a method of extracting a mixture including linear poly(phenylene ether ether ketone) and cyclic poly(phenylene ether ether ketone) using acetone for one week, filtering an acetone extraction soluble component by thermal filtration and cooling down the obtained filtrate for recrystallization. This method also enables recovery of cyclic poly(phenylene ether ether ketone) of high purity. This method, however, requires one week for extraction and has extremely poor efficiency and is accordingly not an industrially available recovery method. Additionally, as specified in this document, the cyclic poly(phenylene ether ether ketone) obtained by this recovery method has a high melting point of 332°C.

Prior Art Documents

Patent Documents:

Patent Document 1: JP S51-119797A
Patent Document 2: JP 2007-302895A
Patent Document 3: JP 2010-70657A
Patent Document 4: WO 2011/081080

Non-Patent Documents:

Non-Patent Document 1: Macromolecules 1996, 29, 5502
Non-Patent Document 2: Macromol. Chem. Phys. 1996, 197, 4069
Non-Patent Document 3: Macromolecules 1993, 26, 2674

Summary of the Invention

Problems to be Solved by the Invention

In order to solve the above problems of the prior art, the object of the present invention is to provide an economical and simple method of efficiently recovering a cyclic poly(phenylene ether ether ketone) composition including a cyclic

poly(phenylene ether ether ketone) mixture of cyclic poly(phenylene ether ether ketone)s having different repeating numbers m.

Means for Solving the Problems

The present invention is accordingly made to solve at least part of the problems described above and may be implemented by the following aspects.

(1) There is provided a recovery method of a cyclic poly(phenylene ether ether ketone) composition, comprising: exposing a mixture including linear poly(phenylene ether ether ketone) and cyclic poly(phenylene ether ether ketone) to a ketone-based solvent and performing solid-liquid separation, so as to recover a cyclic poly(phenylene ether ether ketone) composition including a cyclic poly(phenylene ether ether ketone) mixture of cyclic poly(phenylene ether ether ketone)s which are expressed by General Formula (I) and have different repeating numbers m:

[Chem. 3]

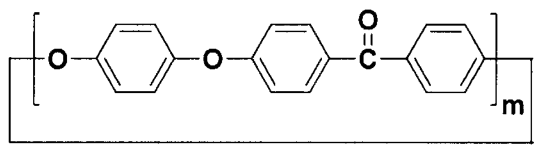

(I)

(wherein m in (I) is an integral number of 2 to 40.)

(2) There is provided the recovery method of the cyclic poly(phenylene ether ether ketone) composition described above in (1), wherein the cyclic poly(phenylene ether ether ketone) mixture is a mixture of cyclic poly(phenylene ether ether ketone)s having three or more different integral numbers as the different repeating numbers m.

(3) There is provided the recovery method of the cyclic poly(phenylene ether ether ketone) composition described above in either (1) or (2), wherein the cyclic poly(phenylene ether ether ketone) mixture is a mixture of cyclic poly(phenylene ether ether ketone)s having three or more consecutive different integral numbers as the different repeating numbers m.

(4) There is provided the recovery method of the cyclic poly(phenylene ether ether ketone) composition described above in any one of (1) to (3), wherein the cyclic poly(phenylene ether ether ketone) composition includes 60% by weight or more of the cyclic poly(phenylene ether ether ketone)s.

(5) There is provided the recovery method of the cyclic poly(phenylene ether ether ketone) composition described above in any one of (1) to (4), wherein the cyclic poly(phenylene ether ether ketone) composition has a melting point of 270°C or lower.

(6) There is provided the recovery method of the cyclic poly(phenylene ether ether ketone) composition described above in any one of (1) to (5), wherein the mixture including the linear poly(phenylene ether ether ketone) and the cyclic poly(phenylene ether ether ketone) is a mixture (a) prepared by reaction under heating of a mixture (x) including a dihalogenated aromatic ketone compound, a dihydroxy aromatic compound, a base and an organic polar solvent.

(7) There is provided the recovery method of the cyclic poly(phenylene ether ether ketone) composition described above in (6), the recovery method exposing the mixture (a) to the ketone-based solvent and performing solid-liquid separation, so as to recover the cyclic poly(phenylene ether ether ketone) composition from an obtained filtrate.

(8) There is provided the recovery method of the cyclic poly(phenylene ether ether ketone) composition described above in any one of (1) to (5), wherein the mixture including the linear poly(phenylene ether ether ketone) and the cyclic poly(phenylene ether ether ketone) is a mixture (b) obtained from an organic solvent (A)-soluble component by preparing a mixture (a) by reaction under heating of a mixture (x) including a dihalogenated aromatic ketone compound, a dihydroxy aromatic compound, a base and an organic polar solvent and exposing the prepared mixture (a) to an organic solvent (A).

(9) There is provided the recovery method of the cyclic poly(phenylene ether ether ketone) composition described above in (8), the recovery method exposing the mixture (b) to the ketone-based solvent, subsequently exposing the mixture (b) to a solvent having low solubility for a poly(phenylene ether ether ketone) component and miscibility with the ketone-based solvent and subsequently performing solid-liquid separation, so as to recover the cyclic poly(phenylene ether ether ketone) composition.

(10) There is provided the recovery method of the cyclic poly(phenylene ether ether ketone) composition described above in either (8) or (9), wherein the organic solvent (A) is a solvent having high solubility for the cyclic poly(phenylene ether ether ketone) and low solubility for the linear poly(phenylene ether ether ketone).

(11) There is provided the recovery method of the cyclic poly(phenylene ether ether ketone) composition described

above in any one of (1) to (10), wherein the ketone-based solvent is acetone and/or methyl ethyl ketone.

(12) There is provided the recovery method of the cyclic poly(phenylene ether ether ketone) composition described above in any one of (1) to (11), wherein the ketone-based solvent is acetone.

(13) There is provided a production method of poly(phenylene ether ether ketone) by thermal ring-opening polymerization of the cyclic poly(phenylene ether ether ketone) composition obtained by the recovery method described above in any one of (1) to (12).

Advantageous Effects

The present invention provides a recovery method of a cyclic poly(phenylene ether ether ketone) composition and more specifically provides a method of efficiently recovering a cyclic poly(phenylene ether ether ketone) composition by an economical and simple technique.

Mode for Carrying Out the Invention

The following describes embodiments of the invention in detail.

(1) Cyclic Poly(Phenylene Ether Ether Ketone)

The cyclic poly(phenylene ether ether ketone) according to an embodiment of the invention is a cyclic compound that has para-phenylene ketone and para-phenylene ether as repeating structural units and is expressed by General Formula (I) given below:

[Chem. 4]

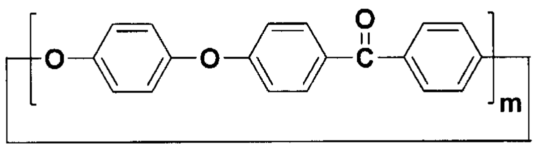

(I)

The number of repetitions m in Formula (I) is an integral number. This number of repetitions m is preferably not less than 2 and not greater than 40. The above number of repetitions m is more preferably not greater than 30, is furthermore preferably not greater than 15 and is especially preferably in a range of not greater than 10. The greater number of repetitions m causes the higher melting point of the cyclic poly(phenylene ether ether ketone). In terms of enabling the cyclic poly(phenylene ether ether ketone) to be fused and melted at low temperature, it is preferable to set the number of repetitions m in the above range.

The cyclic poly(phenylene ether ether ketone) expressed by Formula (I) is preferably a mixture having different numbers of repetitions m, is more preferably a cyclic poly(phenylene ether ether ketone) mixture consisting of cyclic poly(phenylene ether ether ketone)s having at least three or more different numbers of repetitions m, is furthermore preferably a mixture consisting of cyclic poly(phenylene ether ether ketone)s having four or more different numbers of repetitions m and is especially preferably a mixture consisting of cyclic poly(phenylene ether ether ketone)s having five or more different numbers of repetitions m. Additionally, it is specifically preferable that the cyclic poly(phenylene ether ether ketone)s included in the above cyclic poly(phenylene ether ether ketone) mixture have consecutive numbers of repetitions m. Compared with a single compound having a single number of repetitions m, a mixture having different numbers of repetitions m is likely to have a lower melting point. Moreover, compared with a cyclic poly(phenylene ether ether ketone) mixture having two different numbers of repetitions m, a mixture having three or more different number of repetitions m is likely to have an even lower melting point. Furthermore, compared with a mixture having non-consecutive numbers of repetitions m (for example, 3, 5 and 7), a mixture having consecutive numbers of repetitions m (for example, 4, 5 and 6) is likely to have an even lower melting point. The cyclic poly(phenylene ether ether ketone) having each number of repetitions m herein can be analyzed by component separation by high-performance liquid chromatography. Additionally, the composition of the cyclic poly(phenylene ether ether ketone), i.e., the weight fraction of each of the cyclic poly(phenylene ether ether ketone)s having the respective numbers of repetitions m included in the cyclic poly(phenylene ether ether ketone) mixture can be calculated from the peak area ratio of each cyclic poly(phenylene ether ether ketone) by high-performance liquid chromatography.

Furthermore, the cyclic poly(phenylene ether ether ketone) composition according to the embodiment of the

invention has a melting point of not higher than 270°C and is characterized by a significantly lower melting point, compared with linear poly(phenylene ether ether ketone) having a substantially equivalent molecular weight. The melting point is preferably not higher than 250°C and is more preferably not higher than 230°C. The lower melting point of the cyclic poly(phenylene ether ether ketone) composition enables reduction of the processing temperature during processing such as molding. This also enables a lower process temperature to be set in the process of producing a high polymer by using the cyclic poly(phenylene ether ether ketone) composition as a poly(phenylene ether ether ketone) prepolymer, thus reducing the energy required for processing. The melting point of the cyclic poly(phenylene ether ether ketone) composition may be determined by measurement of an endothermic peak temperature using a differential scanning calorimeter.

The cyclic poly(phenylene ether ether ketone) composition according to the embodiment of the invention is a cyclic poly(phenylene ether ether ketone) composition including 60% by weight or more of the cyclic poly(phenylene ether ether ketone). A composition including 65% by weight or more of the cyclic poly(phenylene ether ether ketone) is more preferable; a composition including 70% by weight or more of the cyclic poly(phenylene ether ether ketone) is furthermore preferable; and a composition including 75% by weight or more of the cyclic poly(phenylene ether ether ketone) is especially preferable. The primary impurity component of the cyclic poly(phenylene ether ether ketone) composition, i.e., the component other than the cyclic poly(phenylene ether ether ketone) is linear poly(phenylene ether ether ketone). This linear poly(phenylene ether ether ketone) has a high melting point, so that the higher weight fraction of the linear poly(phenylene ether ether ketone) is likely to provide a cyclic poly(phenylene ether ether ketone) composition having the higher melting point. The cyclic poly(phenylene ether ether ketone) composition having a low melting point can thus be obtained by setting the weight fraction of the cyclic poly(phenylene ether ether ketone) in the cyclic poly(phenylene ether ether ketone) composition to the above range. Additionally, in terms of obtaining the poly(phenylene ether ether ketone) having a sufficiently high degree of polymerization in the process of polymerization using the cyclic poly(phenylene ether ether ketone) composition as a poly(phenylene ether ether ketone) prepolymer, it is preferable that the cyclic poly(phenylene ether ether ketone) in the cyclic poly(phenylene ether ether ketone) composition has the weight fraction in the above range.

The reduced viscosity ($\eta$) of the cyclic poly(phenylene ether ether ketone) composition according to the embodiment of the invention having the characteristics described above is, for example, preferably not higher than 0.1 dL/g, is more preferably not higher than 0.09 dL/g and is furthermore preferably not higher than 0.08 dL/g. Unless otherwise specified, the reduced viscosity according to the embodiment of the invention indicates a value measured by using an Ostwald viscosimeter at 25°C immediately after completion of dissolution in a concentrated sulfuric acid solution having a concentration of 0.1 g/dL (weight of cyclic poly(phenylene ether ether ketone) composition or linear poly(phenylene ether ether ketone)/ volume of 98% by weight of concentrated sulfuric acid) in order to minimize the effect of sulfonation. The calculation of the reduced viscosity follows an equation given below:

$$\eta = \{(t/t0) - 1\} / C$$

(wherein t represents the number of seconds when the sample solution passes through; t0 represents the number of seconds when the solvent (98% by weight of concentrated sulfuric acid) passes through; and C represents the concentration of the solution).

(2) Linear Poly(Phenylene Ether Ether Ketone)

The linear poly(phenylene ether ether ketone) according to an embodiment of the invention is a linear compound that has para-phenylene ketone and para-phenylene ether as repeating structural units and is expressed by General Formula (II) given below:

[Chem. 5]

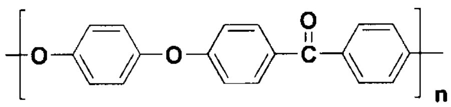 (I I)

The number of repetitions n in Formula (II) is not specifically limited but may be in the range of 10 to 10000, is preferably in the range of 20 to 5000 and is more preferably in the range of 30 to 1000.

The reduced viscosity (η) of the linear poly(phenylene ether ether ketone) according to the embodiment of the invention is not specifically limited. The reduced viscosity (η) of the linear poly(phenylene ether ether ketone) is, however in the range of 0.1 to 2.5 dL/g, is preferably in the range of 0.2 to 2.0 dL/g and is more preferably in the range of 0.3 to 1.8 dL/g. In general, the lower reduced viscosity of the linear poly(phenylene ether ether ketone), i.e, the lower molecular weight of the linear poly(phenylene ether ether ketone), has the higher solubility in an organic polar solvent and thus advantageously reduces the time required for the reaction. The linear poly(phenylene ether ether ketone) having the reduced viscosity in the above range is usable without any essential problem.

(3) Dihalogenated Aromatic Ketone Compound

A dihalogenated aromatic ketone compound used according to an embodiment of the invention is an aromatic ketone compound expressed by General Formula (III):

[Chem. 6]

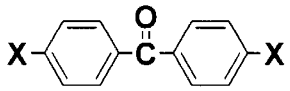
(III)

Herein X in General Formula (III) represents a halogeno group selected from, for example, fluorine, chlorine, bromine, iodine and astatine. Additionally, two halogeno groups included in General Formula (III) may be identical or may be different. Concrete examples of this dihalogenated aromatic ketone compound include 4,4'-difluorobenzophenone, 4,4'-dichlorobenzophenone, 4,4'-dibromobenzophenone, 4,4'-diiodobenzophenone, 4-fluoro-4'-chlorobenzophenone, 4-fluoro-4'-bromobenzophenone, 4-fluoro-4'-iodobenzophenone, 4-chloro-4'-bromobenzophenone, 4-chloro-4'-iodobenzophenone and 4-bromo-4'-idodobenzophenone. Among these compounds, 4,4'-difluorobenzophenone is specified as a preferable concrete example in terms of the reactivity, and 4,4'-dichlorobenzophenone is specified as a preferable concrete examples in terms of the economic efficiency. Especially preferable concrete example is 4,4'-difluorobenzophenone. A single compound may be used as this dihalogenated aromatic ketone compound, or a mixture of two or more compounds may be used as the dihalogenated aromatic ketone compound.

(4) Dihydroxy Aromatic Compound

A dihydroxy aromatic compound used according to an embodiment of the invention is an aromatic compound expressed by General Formula (IV):

[Chem. 7]

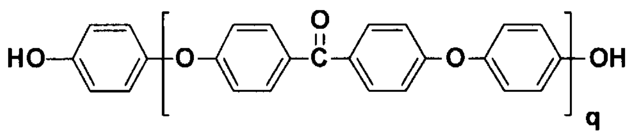
(IV)

The number of repetitions q in General Formula (IV) herein is not specifically limited, but hydroquinone having q=0 is specified as a preferable concrete example. The upper limit of the number of repetitions q in General Formula (IV) is not specifically restricted, but a dihydroxy aromatic compound having q=2 or below is specified as a preferable dihydroxy aromatic compound.

The used amount of this dihydroxy aromatic compound is preferably in the range of 0.8 to 1.2 mol, is more preferably in the range of 0.9 to 1.1 mol, is furthermore preferably in the range of 0.95 to 1.05 mol and is especially preferably in the range of 0.98 to 1.03 mol, relative to 1.0 mol of the dihalogenated aromatic ketone compound. Controlling the used amount of the dihydroxy aromatic compound in the above preferable range can suppress the decomposition reaction of the produced cyclic poly(phenylene ether ether ketone) and is also likely to suppress production of the linear poly(phenylene ether ether ketone), which is difficult to be separated from the cyclic poly(phenylene ether ether ketone).

(5) Base

Available examples of a base used according to an embodiment of the invention include: carbonates of alkali

metals such as lithium carbonate, sodium carbonate, potassium carbonate, rubidium carbonate and cesium carbonate; carbonates of alkaline earth metals such as calcium carbonate, strontium carbonate and barium carbonate; bicarbonates of alkali metals such as lithium hydrogen carbonate, sodium hydrogen carbonate, potassium hydrogen carbonate, rubidium hydrogen carbonate and cesium hydrogen carbonate; bicarbonates of alkaline earth metals such as calcium hydrogen carbonate, strontium hydrogen carbonate and barium hydrogen carbonate; hydroxides of alkali metals such as lithium hydroxide, sodium hydroxide, potassium hydroxide, rubidium hydroxide and cesium hydroxide; and hydroxides of alkaline earth metals such as calcium hydroxide, strontium hydroxide and barium hydroxide. Among them, in terms of the easiness of handling and the reactivity, carbonates such as sodium carbonate and potassium carbonate and bicarbonates such as sodium hydrogen carbonate and potassium hydrogen carbonate are preferable; sodium carbonate and potassium carbonate are more preferable; and potassium carbonate is furthermore preferable. Any of these bases may be used alone, or two or more of these bases may be used as a mixture. Any of these bases is preferably used in the form of an anhydride but may also be used as a hydrate or an aqueous mixture. The aqueous mixture herein indicates an aqueous solution, a mixture of an aqueous solution and a solid component or a mixture of water and a solid component.

(6) Organic Polar Solvent

An organic polar solvent used according to an embodiment of the invention is not specifically limited but may be any organic polar solvent which does not substantially interfere with the reaction and does not substantially cause an undesired side reaction, such as degradation of the produced cyclic poly(phenylene ether ether ketone). Concrete examples of such an organic polar solvent include: nitrogen-containing polar solvents such as N-methyl-2-pyrrolidone (NMP), N-methylcaprolactam, N,N-dimethylformamide (DMF), N-N-dimethylacetamide (DMAc), 1,3-dimethyl-2-imidazolidinone (DMI), hexamethylphosphoramide and tetramethylurea; sulfoxide and sulfone-based solvents such as dimethylsulfoxide (DMSO), dimethyl sulfone, diphenylsulfone and sulfolane; nitrile-based solvents such as benzonitrile; diaryl ethers such as diphenyl ether; ketones such as benzophenone and acetophenone; and mixtures thereof. Any of these organic polar solvents has the high stability of the reaction and is thus preferably used. Among them, preferable are N-methyl-2-pyrrolidone and dimethylsulfoxide; and especially preferable is N-methyl-2-pyrrolidone. These organic polar solvents have excellent stability in a high temperature range and are also preferable in terms of the availability.

(7) Ketone-Based Solvent

According to an embodiment of the invention, a mixture including the linear poly(phenylene ether ether ketone) and the cyclic poly(phenylene ether ether ketone) is exposed to a ketone-based solvent and is subjected to solid-liquid separation, so that the cyclic poly(phenylene ether ether ketone) composition is recovered.

The ketone-based solvent used herein may be, for example, acetone, methyl ethyl ketone, methyl isobutyl ketone, diisobutyl ketone or cyclohexanone. In terms of the easiness of handling, acetone and methyl ethyl ketone are preferable; and in terms of the availability, acetone is especially preferable used. Any of these ketone-based solvents may be used alone, or two or more of ketone-based solvents may be used as a mixture.

(8) Mixture Including Linear Poly(Phenylene Ether Ether Ketone) and Cyclic Poly(Phenylene Ether Ether Ketone)

The present invention provides a recovery method of a cyclic poly(phenylene ether ether ketone) composition, which exposes a mixture including the linear poly(phenylene ether ether ketone) and the cyclic poly(phenylene ether ether ketone) to the ketone based solvent and performs solid-liquid separation.

The mixture used herein may be unquestionably any mixture including the linear poly(phenylene ether ether ketone) and the cyclic poly(phenylene ether ether ketone). A preferable aspect of the mixture is a mixture (a) prepared by reaction under heating of a mixture (x) including the dihalogenated aromatic ketone compound, the dihydroxy aromatic compound, the base and the organic polar solvent. Another preferable aspect is a mixture (b) obtained from an organic solvent (A)-soluble component by exposing the mixture (a) to an organic solvent (A). The following describes the mixtures of these preferable aspects in detail.

(8)-1. Mixture (a)

The mixture including the linear poly(phenylene ether ether ketone) and the cyclic poly(phenylene ether ether ketone) may be a mixture (a) prepared by reaction under heating of a mixture (x) including the dihalogenated aromatic ketone compound, the dihydroxy aromatic compound, the base and the organic polar solvent.

The amount of the organic polar solvent in the mixture (x) is not specifically limited but is preferably not less than 0.1 liters, is more preferably not less than 0.15 liters, is furthermore preferably not less than 0.3 liters and is especially preferably not less than 0.5 liters, relative to 1.0 mole of the benzene ring component in the mixture (x). The upper limit

of the amount of the organic polar solvent in the mixture is not specifically restricted but is preferably not greater than 100 liters, is more preferably not greater than 50 liters, is furthermore preferably not greater than 30 liters and is especially preferably not greater than 20 liters, relative to 1.0 mole of the benzene ring component in the mixture. Increasing the amount of the organic polar solvent is likely to improve the selectivity in production of the cyclic poly(phenylene ether ether ketone). The amount of the organic polar solvent greater than 100 liters, however, is likely to reduce the amount of production of the cyclic poly(phenylene ether ether ketone) per unit volume of a reaction vessel and is also likely to increase the time required for the reaction. The amount of the organic polar solvent less than 0.1 liters, on the other hand, decreases the selectivity in production of the cyclic poly(phenylene ether ether ketone). Accordingly, in terms of satisfying both the selectivity and the productivity in production of the cyclic poly(phenylene ether ether ketone), the organic polar solvent is preferably used in the above range. The amount of the organic polar solvent herein is indicated by a volume of the solvent at ordinary temperatures and ordinary pressures. The used amount of the organic polar solvent in the mixture (x) is the amount calculated by subtracting the amount of the organic polar solvent expelled out of the reaction system by, for example, dehydration operation from the amount of the organic polar solvent introduced into the reaction system. The benzene ring component in the mixture (x) herein means a benzene ring component included in the raw material which can become the structural component of the cyclic poly(phenylene ether ether ketone) by the reaction. The "molar number" of the benzene ring component in these raw materials indicates the "number of benzene rings constituting the compound". For example, 1 mol of 4,4'-difluorobenzophenone corresponds to 2 mol of the benzene ring component, and 1 mol of hydroquinone corresponds to 1 mol of the benzene ring component. Accordingly, a mixture including 1 mol of 4,4'-difluorobenzophenone and 1 mol of hydroquinone corresponds to 3 mol of the benzene ring component. The benzene ring component that cannot become the structural component of the cyclic poly(phenylene ether ether ketone) by the reaction, for example, toluene, is regarded as 0 mol of the benzene ring component.

The used amount of the base in the mixture (x) is preferably not less than an equivalent as the stoichiometric ratio to the dihydroxy aromatic compound. On the assumption that the used amount of a divalent base such as sodium carbonate or potassium carbonate is Y mol and the used amount of a monovalent base such as sodium hydrogen carbonate or potassium hydrogen carbonate is Z mol, for example, the concrete used amount of the base expressed as (Y+2Z) is preferably in the range of 1.0 to 1.10 mol, is more preferably in the range of 1.00 mol to 1.05 mol and is furthermore preferably in the range of 1.00 mol to 1.03 mol relative to 1.0 mol of the dihydroxy aromatic compound used for manufacture of the mixture (a).

The reason why the stoichiometric ratio is preferable as described above is theoretically attributed to the following. The used amount of the base in this range enables sufficient production of a metal salt of the dihydroxy aromatic compound. Additionally, this can also suppress the progress of an undesired reaction, such as decomposition reaction of cyclic poly(phenylene ether ether ketone), caused by a large excess of the base.

A metal salt of the dihydroxy aromatic compound separately prepared from the dihydroxy aromatic compound and the base may also be used. In this application, an excess amount of the base may be supplied by adding a desired base described above. The excess amount of the base supplied expressed as (Y+2Z) is preferably in the range of 0 to 0.10 mol, is more preferably in the range of 0 to 0.05 mol and is furthermore preferably in the range of 0 to 0.03 mol relative to 1.0 mol of the dihydroxy aromatic compound used for manufacture of the mixture (a). Controlling the excess amount of the base in the preferable range can also suppress the progress of an undesired reaction, such as decomposition reaction of cyclic poly(phenylene ether ether ketone).

The reaction temperature in the reaction under heating of the mixture (x) including the dihalogenated aromatic ketone compound, the dihydroxy aromatic compound, the base and the organic polar solvent differs according to the types and the amounts of the materials included in the mixture (x) and is thus not unequivocally specifiable, but is generally not lower than 120°C, is preferably not lower than 150°C and is more preferably not lower than 200°C. This reaction temperature is also not higher than 350°C, is preferably not higher than 330°C and is more preferably not higher than 320°C. The reaction temperature in this range is likely to provide the high reaction rate. The reaction herein may be any of a single-stage reaction that maintains a constant temperature, a multi-stage reaction that raises the temperature stepwise and a reaction that changes the temperature continuously.

The reaction time depends on the types and the amounts of the materials included in the mixture (x) or the reaction temperature and is thus not unequivocally specifiable. The reaction time is, however, preferably not shorter than 0.1 hours, is more preferably not shorter than 0.5 hours and is furthermore preferably not shorter than 1 hour. The upper limit of the reaction time is, on the other hand, not specifically restricted. The reaction sufficiently proceeds in not longer than 40 hours, and the reaction time employed may be preferably not longer than 10 hours and is more preferably not longer than 6 hours. Controlling the reaction time in this range is likely to sufficiently reduce the unreacted raw material components.

In the reaction under heating of the mixture (x), a component which does not significantly interfere with the reaction or a component which has the effect of accelerating the reaction may be added to the mixture (x), in addition to the above essential components. The means of the reaction are not specifically limited, but it is preferable that the

reaction proceeds under the stirring condition. Additionally, any of known various polymerization systems and reaction systems, such as batch system or continuous system, may be employed. The atmosphere in manufacture is desirably a non-oxidizing atmosphere. The manufacture is preferably performed under an inert atmosphere such as nitrogen, helium or argon. In terms of the economic efficiency and the easiness of handling, the manufacture is more preferably performed under nitrogen atmosphere.

With respect to the above reaction, the presence of a large amount of water in the reaction system is likely to reduce the reaction rate of the reaction and produce a byproduct which is difficult to be separated from the cyclic poly(phenylene ether ether ketone). Accordingly, the water content present in the system during the reaction is preferably not higher than 3.0% by weight, is more preferably not higher than 1.0% by weight, is furthermore preferably not higher than 0.5% by weight and is especially preferably not higher than 0.3% by weight.

The water in the case of using a hydrate or an aqueous mixture as the base or the water produced as the byproduct of the reaction may be expelled out of the reaction system as needed basis, so that the water content is set in the range of not higher than this preferable value. The water content present in the system herein is expressed by the weight fraction relative to the total weight of the mixture (x). The water content may be measured by the Karl Fischer method.

The timing of the dehydration operation is not specifically limited but is preferable (i) after mixing the essential components or (ii) after mixing the essential components other than the dihalogenated aromatic ketone compound. When the dehydration operation is performed after mixing the essential components other than the dihalogenated aromatic ketone compound at the above timing (ii), the mixture (a) is manufactured by adding the dihalogenated aromatic ketone compound or by adding the dihalogenated aromatic ketone compound and the organic polar solvent after the dehydration operation.

The method of removing water may be any method that can expel water out of the reaction system; for example, the method of dehydration by high temperature heating or the method by azeotropic distillation using an azeotropic solvent. In terms of the dehydration efficiency, the method by azeotropic distillation is preferable.

The azeotropic solvent used for azeotropic distillation herein may be any organic compound which can form an azeotropic mixture with water, wherein the boiling point of the azeotropic mixture is lower than the boiling point of the organic polar solvent used for the reaction. Concrete examples of the azeotropic solvent include: hydrocarbon-based solvents such as hexane, cyclohexane, heptane, benzene, toluene and xylene; and inactive chlorinated aromatic compounds such as chlorobenzene and dichlorobenzene. Among them, toluene and xylene are preferable. The amount of the azeotropic solvent required for formation of the azeotropic mixture with water differs according to the amount of water present in the system and the type of the solvent and is thus not unequivocally specifiable. It is, however, preferable to use an excess amount of the azeotropic solvent that is greater than the required amount for removing the water present in the reaction system.

The amount of the azeotropic solvent is preferably not less than 0.2 liters, is more preferably not less than 0.5 liters and is furthermore preferably not less than 1.0 liter, relative to 1.0 mol of the dihydroxy aromatic compound in the mixture (x). The upper limit of the amount of the azeotropic solvent is, on the other hand, not specifically restricted but is preferably not greater than 20.0 liters, is more preferably not greater than 10.0 liters and is furthermore preferably not greater than 5.0 liters, relative to 1.0 mol of the dihydroxy aromatic compound in the mixture. The excessively large amount of the azeotropic solvent, however, lowers the polarity of the mixture and is thereby likely to reduce the efficiency of the reaction of the base with the dihydroxy aromatic compound. The amount of the azeotropic solvent herein is expressed by the volume of the solvent at ordinary temperatures and ordinary pressures.

When azeotropic distillation of water is performed by using the principle of a Dean-Stark apparatus, the amount of the azeotropic solvent in the reaction system can be continuously maintained constant, so that the used amount of the azeotropic solvent can be further reduced.

The temperature for expelling water out of the reaction system is not unequivocally specifiable, since the boiling point of the azeotropic mixture with water differs according to the type of the azeotropic solvent. The temperature is preferably not lower than the boiling point of the azeotropic mixture with water but not higher than the boiling point of the organic polar solvent used for the reaction. More specifically, the temperature for expelling water out of the reaction system may be in the range of 60 to 170°C, is preferably in the range of 80 to 170°C, is more preferably in the range of 100 to 170°C and is furthermore preferably in the range of 120 to 170°C. The method of removing water may be any of a method that maintains a constant temperature, a method that raises the temperature stepwise and a temperature that changes the temperature continuously, in the desired temperature range. Additionally, it is also preferable to perform the above azeotropic distillation under reduced pressure. The azeotropic distillation under reduced pressure is likely to remove water with the higher efficiency.

It is preferable to expel the above azeotropic solvent out of the system after the azeotropic distillation. The timing of expelling the azeotropic solvent out of the system is preferably after completion of the azeotropic distillation of water. When the dehydration operation is performed after mixing the essential components other than the dihalogenated aromatic ketone compound at the above timing (ii), it is preferable to remove the azeotropic solvent at the stage prior

to addition of the dihalogenated aromatic ketone compound or at the stage prior to addition of the dihalogenated aromatic ketone compound and the organic polar solvent. A large amount of the azeotropic solvent remaining in the system lowers the polarity of the reaction system and is likely to reduce the reaction rate of production of the cyclic poly(phenylene ether ether ketone). It is accordingly desired to perform the removal operation of the azeotropic solvent.

The amount of the azeotropic solvent remaining in the system during the production reaction of the cyclic poly(phenylene ether ether ketone) is preferably not greater than 20%, is more preferably not greater than 10%, is furthermore preferably not greater than 8% and is especially preferably not greater than 6%, relative to the amount of the organic polar solvent in the mixture (x). It is preferable to remove the azeotropic solvent, such that the amount of the azeotropic solvent is in this range.

The method of removing the azeotropic solvent is preferably a method by distillation and may use an inert gas, such as nitrogen, helium or argon, as the carrier gas. Another preferable method performs distillation under reduced pressure. This is likely to remove the azeotropic solvent with the higher efficiency.

The temperature for removing the azeotropic solvent may be any temperature that can expel the azeotropic solvent out of the reaction system. More specifically, the temperature for removing the azeotropic solvent is in the range of 60 to 170°C, is preferably in the range of 100 to 170°C, is more preferably in the range of 120 to 170°C and is furthermore preferably in the range of 140 to 170°C. The method of removing the azeotropic solvent may be any of a method that maintains a constant temperature in the desired temperature range, a method that raises the temperature stepwise and a temperature that changes the temperature continuously.

The reaction mixture thus obtained includes at least the cyclic poly(phenylene ether ether ketone), the linear poly(phenylene ether ether ketone) and the organic polar solvent. Byproduct salts may also be included as another component.

This reaction mixture is used as the mixture (a) in the recovery method of the cyclic poly(phenylene ether ether ketone) composition according to the embodiment of the invention. A mixture which includes the linear poly(phenylene ether ether ketone) and the cyclic poly(phenylene ether ether ketone) and is obtained by purification treatment described below may alternative be used as the mixture (a).

The method of purification treatment of the reaction mixture obtained by the above preferable preparation method is not specifically limited. For example, one available method exposes the reaction mixture to a solvent after removal of part or bulk of the organic polar solvent by an operation such as distillation, so as to recover a solid mixture of the linear poly(phenylene ether ether ketone) and the cyclic poly(phenylene ether ether ketone). The solvent used has low solubility for the poly(phenylene ether ether ketone) component, miscibility with the organic polar solvent and solubility for the byproduct salts. The solvent having these characteristics is generally a solvent of relatively high polarity. The preferable solvent differs according to the type of the organic polar solvent used and the types of the byproduct salts, but may be, for example, water or alcohols such as methanol, ethanol, propanol, isopropyl alcohol, butanol or hexanol. In terms of the availability and the economic efficiency, water, methanol and ethanol are preferable, and water is especially preferable.

Such purification treatment can reduce the amount of the organic polar solvent and the amount of the byproduct salts contained in the solid mixture of the cyclic poly(phenylene ether ether ketone) and the linear poly(phenylene ether ether ketone). This purification treatment enables both the cyclic poly(phenylene ether ether ketone) and the linear poly(phenylene ether ether ketone) to precipitate as solid components. A mixture of the cyclic poly(phenylene ether ether ketone) and the linear poly(phenylene ether ether ketone) can thus be recovered by a known solid-liquid separation method. This is likely to further reduce the amount of the organic polar solvent and the amount of the byproduct salts contained in the solid mixture of the cyclic poly(phenylene ether ether ketone) and the linear poly(phenylene ether ether ketone).

The above purification treatment may be performed with stirring or under heating as appropriate. The temperature of the purification treatment is not specifically limited but is preferably in the range of 20 to 220°C and is more preferably in the range of 50 to 200°C. These temperatures facilitate removal of the byproduct salts and enable the purification treatment to be performed at relatively low pressure.

When water is used as the solvent, the water is preferably distilled water or deionized water but may be used in the form of an aqueous solution including an acidic organic compound such as formic acid, acetic acid, propionic acid, butyric acid, chloroacetic acid, dichloroacetic acid, acrylic acid, crotonic acid, benzoic acid, salicylic acid, oxalic acid, malonic acid, succinic acid, phthalic acid or fumaric acid or its alkali metal salt or alkaline earth metal salt. Also usable is an aqueous solution including an acidic inorganic compound such as sulfuric acid, phosphoric acid, hydrochloric acid, carbonic acid or silicic acid or an ammonium ion. When the solid mixture of the cyclic poly(phenylene ether ether ketone) and the linear poly(phenylene ether ether ketone) obtained by this purification treatment contains the solvent, the solvent may be optionally removed by drying.

It is preferable to use the mixture which includes the linear poly(phenylene ether ether ketone) and the cyclic poly(phenylene ether ether ketone) and is obtained by the above purification treatment, as the mixture (a) in the recovery method of the cyclic poly(phenylene ether ether ketone) composition according to the invention.

(8)-2. Mixture (b)

The mixture including the linear poly(phenylene ether ether ketone) and the cyclic poly(phenylene ether ether ketone) may be a mixture (b) obtained from an organic solvent (A)-soluble component by exposing the above mixture (a) to an organic solvent (A).

The organic solvent (A) is not specifically limited but may be any organic solvent that has dissolution performance to dissolve 50% by weight or more of the cyclic poly(phenylene ether ether ketone) present in the mixture (a). The organic solvent (A) is, however, preferably a solvent having high solubility for the cyclic poly(phenylene ether ether ketone) but low solubility for the linear poly(phenylene ether ether ketone). The solubility of the organic solvent is affected by various factors, such as the temperature and the pressure during dissolution and the used amount. The organic solvent (A) is not specifically limited, but any organic solvent satisfying the above conditions is selectable. In terms of recovery of the cyclic poly(phenylene ether ether ketone) composition with the higher efficiency by the simple operation, it is desirable to dissolve a large amount of the cyclic poly(phenylene ether ether ketone) by a small used amount of the organic solvent. Examples of the solvent having such characteristic include: hydrocarbon solvents such as pentane, hexane, heptane, octane, cyclohexane, cyclopentane, benzene, toluene and xylene; halogenated solvents such as chloroform, bromoform, methylene chloride, 1,2-dichloroethane, 1,1,1-trichloroethane, chlorobenzene and 2,6-dichlorotoluene; N-alkyl pyrrolidones such as N-methyl-2-pyrrolidone, N-ethyl-2-pyrrolidone and N-cyclohexyl-2-pyrrolidone; caprolactams such as N-methyl-ε-caprolactam and ε-caprolactam; aprotic solvents such as 1,3-dimethyl-2-imidazolidinone, N,N-dimethylacetamide, N,N-dimethylformamide and triamide hexamethylphosphate; and the above ketone-based solvents.

In general, the linear poly(phenylene ether ether ketone) has high crystallinity and extremely low solubility in a solvent. The cyclic poly(phenylene ether ether ketone) and the linear poly(phenylene ether ether ketone) have significantly different solubilities in the solvent, and generally the cyclic poly(phenylene ether ether ketone) has the higher solubility in the solvent. The operation of exposing the mixture (a) to the organic solvent (A) can thus provide a mixture including the linear poly(phenylene ether ether ketone) and the cyclic poly(phenylene ether ether ketone) and having a large content of the cyclic poly(phenylene ether ether ketone).

The pressure in the reaction system in the process of exposing the mixture (a) to the organic solvent (A) is not specifically limited, but is preferably ordinary pressure or slightly pressurized and is especially preferably ordinary pressure. The reaction system under such pressure has the advantage that structural members of a reactor are inexpensive. From this point of view, it is preferable to avoid a pressurized condition that requires an expensive pressure vessel as the pressure in the reaction system. Exposing the mixture (a) to the organic solvent (A) in such pressure of the reaction system is likely to enable extraction and recovery of the cyclic poly(phenylene ether ether ketone) in the mixture (a) with high efficiency.

The atmosphere in which the mixture (a) is exposed to the organic solvent (A) is not specifically limited but is preferably a non-oxidizing atmosphere. It is preferable to perform the exposure under an inert gas atmosphere such as nitrogen, helium or argon. Among them, in terms of the economic efficiency and the easiness of handling, it is especially preferable to perform the exposure under nitrogen atmosphere.

The temperature at which the mixture (a) is exposed to the organic solvent (A) is not specifically limited. In general, the higher temperature is likely to accelerate dissolution of the cyclic poly(phenylene ether ether ketone) included in the mixture (a), in the organic solvent (A). For example, 20 to 150°C is thus specified as the available temperature range.

The time when the mixture (a) is exposed to the organic solvent (A) differs according to the type of the organic solvent (A) used and the temperature and is thus not unequivocally specifiable but may be, for example 1 minute to 50 hours. This ranges is likely to enable the cyclic poly(phenylene ether ether ketone) to be sufficiently dissolved in the organic solvent (A).

The method of exposing the mixture (a) to the organic solvent (A) is not specifically limited, but any of known general techniques may be employed. Available methods include: for example, a method that mixes the mixture (a) with the organic solvent (A) optionally with stirring and subsequently recovers the soluble part; a method that sprays the organic solvent (A) onto the mixture (a) on any of various filters and simultaneously dissolves the cyclic poly(phenylene ether ether ketone) in the organic solvent (A); and a method that employs the principle of the Soxhlet extraction method.

The used amount of the organic solvent (A) during exposure of the mixture (a) to the organic solvent (A) is not specifically limited but may be in a range of 0.5 to 100 [L/kg] as the liquor ratio to the weight of the mixture (a). The liquor ratio in this range is likely to facilitate the above mixture (a) to be homogeneously mixed with the organic solvent (A) and also facilitate the cyclic poly(phenylene ether ether ketone) to be sufficiently dissolved in the organic solvent (A). The larger liquor ratio is generally advantageous for dissolution of the cyclic poly(phenylene ether ether ketone) in the organic solvent (A). The excessively large liquor ratio is, however, not expected to have any further effects but may, on the contrary, cause economical disadvantage due to an increase in used amount of the solvent. In the case of repeating the exposure of the mixture (a) to the organic solvent (A), even the small liquor ratio often achieves the sufficient effects. The Soxhlet extraction method has the similar effects in principle and thus often allows even the small liquor ratio to achieve the sufficient effects.

When a solution with the cyclic poly(phenylene ether ether ketone) dissolved therein is obtained in the form of a solid-liquid slurry including the solid form of the linear poly(phenylene ether ether ketone) after the exposure of the mixture (a) to the organic solvent (A), it is preferable to recover the mixture (b) by a known solid-liquid separation method. The solid-liquid separation method may be, for example, separation by filtration, centrifugal separation or decantation. When the cyclic poly(phenylene ether ether ketone) still remains in the solid component, the exposure to the organic solvent (A) and the recovery of the solution may be repeatedly performed to obtain the mixture (b) with no substantial loss of the cyclic poly(phenylene ether ether ketone).

The mixture thus obtained includes at least the cyclic poly(phenylene ether ether ketone), the linear poly(phenylene ether ether ketone) and the organic solvent (A). This mixture may be used as the mixture (b) in the recovery method of the cyclic poly(phenylene ether ether ketone) composition according to the embodiment of the invention. A mixture which includes the linear poly(phenylene ether ether ketone) and the cyclic poly(phenylene ether ether ketone) and is obtained by purification treatment described below may alternative be used as the mixture (b).

The method of purification treatment of the above mixture (b) is not specifically limited. For example, after removal of part or bulk of the organic solvent (A) by an operation such as distillation, an available method exposes the mixture (b) to a solvent which has low solubility for the poly(phenylene ether ether ketone) component and miscibility with the organic solvent (A), optionally under heating, so as to recover a solid mixture of the cyclic poly(phenylene ether ether ketone) and the linear poly(phenylene ether ether ketone). The solvent having these characteristics is generally a solvent of relatively high polarity. The preferable solvent differs according to the type of the organic solvent (A) used and is thus not specifiable. Available examples include water and alcohols such as methanol, ethanol, propanol, isopropyl alcohol, butanol and hexanol. In terms of the availability and the economic efficiency, water, methanol and ethanol are preferable, and water is especially preferable.

Such purification process with the solvent can reduce the amount of the organic solvent (A) contained in the solid mixture of the cyclic poly(phenylene ether ether ketone) and the linear poly(phenylene ether ether ketone). This purification treatment enables both the cyclic poly(phenylene ether ether ketone) and the linear poly(phenylene ether ether ketone) to precipitate as solid components. A mixture of the cyclic poly(phenylene ether ether ketone) and the linear poly(phenylene ether ether ketone) can thus be recovered by a known solid-liquid separation method. This is likely to further reduce the amount of the organic solvent (A) contained in the solid mixture of the cyclic poly(phenylene ether ether ketone) and the linear poly(phenylene ether ether ketone).

The above purification treatment with the solvent may be performed with stirring or under heating as appropriate. The temperature of the purification treatment with the solvent is not specifically limited but is preferably in the range of 20 to 220°C and is more preferably in the range of 50 to 200°C. This temperature range preferably enables the purification treatment, for example, in the state of relatively low pressure. When water is used as the solvent, the water is preferably distilled water or deionized water but may be used in the form of an aqueous solution including an acidic organic compound such as formic acid, acetic acid, propionic acid, butyric acid, chloroacetic acid, dichloroacetic acid, acrylic acid, crotonic acid, benzoic acid, salicylic acid, oxalic acid, malonic acid, succinic acid, phthalic acid or fumaric acid or its alkali metal salt or alkaline earth metal salt. Also usable is an aqueous solution including an acidic inorganic compound such as sulfuric acid, phosphoric acid, hydrochloric acid, carbonic acid or silicic acid or an ammonium ion. When the solid mixture of the cyclic poly(phenylene ether ether ketone) and the linear poly(phenylene ether ether ketone) obtained after this treatment contains the solvent used for the purification treatment, the solvent may be optionally removed by drying.

It is preferable to use the mixture which includes the linear poly(phenylene ether ether ketone) and the cyclic poly(phenylene ether ether ketone) and is obtained by the above purification treatment, as the mixture (b) in the recovery method of the cyclic poly(phenylene ether ether ketone) composition according to the embodiment of the invention.

(9) Recovery Method of Cyclic Poly(Phenylene Ether Ether Ketone) Composition

According to an embodiment of the invention, the mixture including the linear poly(phenylene ether ether ketone) and the cyclic poly(phenylene ether ether ketone) is exposed to the ketone-based solvent and is subjected to solid-liquid separation, so that the cyclic poly(phenylene ether ether ketone) composition is recovered.

The pressure in the reaction system in the process of exposing the above mixture to the ketone-based solvent is preferably ordinary pressure or slightly pressurized and is especially preferably ordinary pressure. The reaction system under such pressure has the advantage that structural members of a reactor are inexpensive. From this point of view, it is preferable to avoid a pressurized condition that requires an expensive pressure vessel as the pressure in the reaction system.

The temperature at which the mixture is exposed to the ketone-based solvent is not specifically limited. As described above, however, it is preferable to expose the mixture including the linear poly(phenylene ether ether ketone) and the cyclic poly(phenylene ether ether ketone) to the ketone-based solvent under ordinary pressure. The upper limit temperature is thus preferably the reflex temperature of the used solvent under atmospheric pressure. In the case of using the preferable ketone-based solvent described above, for example, 20 to 160°C is specified as the concrete

temperature range.

The time when the mixture is exposed to the ketone-based solvent differs according to the type of the ketone-based solvent used and the temperature and is not unequivocally specifiable but may be, for example, 1 minute to 50 hours. This range is likely to enable the cyclic poly(phenylene ether ether ketone) to be efficiently recovered.

The method of exposing the above mixture to the ketone-based solvent is not specifically limited, but any of known general techniques may be employed. For example, an available method mixes the mixture with the ketone-based solvent, optionally with stirring. The used amount of the ketone-based solvent during exposure of the mixture to the ketone-based solvent is not specifically limited but may be in a range of 0.5 to 100 as the liquor ratio to the weight of the mixture. The liquor ratio in this range is likely to facilitate the mixture to be mixed with the ketone-based solvent. The larger liquor ratio is generally advantageous for recovery of the cyclic poly(phenylene ether ether ketone) composition. The excessively large liquor ratio is, however, not expected to have any further effects but may, on the contrary, cause economical disadvantage due to an increase in used amount of the ketone-based solvent. In the case of repeating the exposure of the mixture to the ketone-based solvent, even the small liquor ratio often achieves the sufficient effects and often enables recovery of the cyclic poly(phenylene ether ether ketone) composition with high efficiency.

When the above mixture (a) is used as the mixture including the linear poly(phenylene ether ether ketone) and the cyclic poly(phenylene ether ether ketone), poly(phenylene ether ether ketone), it is preferable to separate a component insoluble in the ketone-based solvent by a known solid-liquid separation method, optionally under heating, after exposure to the ketone-based solvent. The solid-liquid separation operation after exposure to the ketone-based solvent is likely to recover the cyclic poly(phenylene ether ether ketone) composition of high purity from the filtrate. The solid-liquid separation method may be, for example, separation by filtration, centrifugal separation or decantation. When the cyclic poly(phenylene ether ether ketone) remains in the solid component, the exposure to the ketone-based solvent and the solid-liquid separation operation may be repeatedly perform to reduce the loss of the cyclic poly(phenylene ether ether ketone).

According to an embodiment of the invention, the ketone-based solvent is removed from the obtained mixture including at least the cyclic poly(phenylene ether ether ketone) composition and the ketone-based solvent, so that the cyclic poly(phenylene ether ether ketone) composition is recovered in the form of a solid component.

The method of removing the ketone-based solvent and recovering the cyclic poly(phenylene ether ether ketone) composition herein is not specifically limited. The method of removal of the ketone-based solvent may be, for example, a method of heating and treating the ketone-based solvent under ordinary pressure or under reduced pressure or a method using a membrane for removal of the ketone-based solvent.

Moreover, another available method may remove part or bulk of the ketone-based solvent as appropriate by an operation such as distillation, expose the mixture to a solvent, optionally under heating, to provide the cyclic poly(phenylene ether ether ketone) composition in the form of a solid component, and recover the cyclic poly(phenylene ether ether ketone) composition by a solid-liquid separation operation. This recovery method is desirable when the above mixture (b) is used as the mixture including the linear poly(phenylene ether ether ketone) and the cyclic poly(phenylene ether ether ketone). The solid-liquid separation method employed herein may be any of known solid-liquid separation techniques and may be, for example, separation by filtration, centrifugal separation or decantation.

The above solvent has the characteristics of low solubility for the poly(phenylene ether ether ketone) component and miscibility with the ketone-based solvent. This solvent is generally a solvent of relatively high polarity. The preferable solvent differs according to the type of the ketone-based solvent used and is not specifiable. Available examples include water and alcohols such as methanol, ethanol, propanol, isopropyl alcohol, butanol and hexanol. In terms of the availability and the economic efficiency, water, methanol and ethanol are preferably used. In recovery of the cyclic poly(phenylene ether ether ketone) composition by this method, the less amount of this solvent added to the ketone-based solvent is likely to increase the purity of the recovered poly(phenylene ether ether ketone) composition.

The above treatment with the solvent may be performed with stirring or under heating as appropriate. The temperature of the treatment with the solvent is not specifically limited but is preferably in the range of 20 to 220°C and is more preferably in the range of 50 to 200°C. This temperature range preferably enables the treatment in the state of relatively low pressure.

When water is used as the solvent, the water is preferably distilled water or deionized water but may be used in the form of an aqueous solution including an acidic organic compound such as formic acid, acetic acid, propionic acid, butyric acid, chloroacetic acid, dichloroacetic acid, acrylic acid, crotonic acid, benzoic acid, salicylic acid, oxalic acid, malonic acid, succinic acid, phthalic acid or fumaric acid or its alkali metal salt or alkaline earth metal salt. Also usable is an aqueous solution including an acidic inorganic compound such as sulfuric acid, phosphoric acid, hydrochloric acid, carbonic acid or silicic acid or an ammonium ion. When the cyclic poly(phenylene ether ether ketone) composition obtained after this treatment contains the solvent used for the treatment, the solvent may be optionally removed by drying.

The cyclic poly(phenylene ether ether ketone) composition obtained by the method according to the method of the invention has different characteristics from those of the generally obtained linear poly(phenylene ether ether ketone) and has a great deal of potential in industry.

(10) Production Method of Poly(Phenylene Ether Ether Ketone)

The cyclic poly(phenylene ether ether ketone) composition according to the embodiment of the invention may be used as a poly(phenylene ether ether ketone) prepolymer and may be converted to a poly(phenylene ether ether ketone) by thermal ring-opening polymerization. The poly(phenylene ether ether ketone) herein is a linear compound that has para-phenylene ketone and para-phenylene ether as repeating structural units and is expressed by General Formula (II) given above.

The reduced viscosity (η) of the poly(phenylene ether ether ketone) obtained by thermal ring-opening polymerization of the cyclic poly(phenylene ether ether ketone) composition according to the embodiment of the invention is not specifically limited but is preferably in the range of 0.1 to 2.5 dL/g, is more preferably in the range of 0.2 to 2.0 dL/g and is furthermore preferably in the range of 0.3 to 1.8 dL/g.

The heating temperature in the process of converting the cyclic poly(phenylene ether ether ketone) composition into the poly(phenylene ether ether ketone) by thermal ring-opening polymerization is preferably not less than a temperature at which the cyclic poly(phenylene ether ether ketone) composition is melted and fused. There is no specific limitation in this temperature condition. When the heating temperature is less than the melting point of the cyclic poly(phenylene ether ether ketone), it takes a long time to obtain the poly(phenylene ether ether ketone) by thermal ring-opening polymerization. Otherwise the thermal ring-opening polymerization does not proceed, so that it is unlikely to obtain poly(phenylene ether ether ketone).

The temperature at which the cyclic poly(phenylene ether ether ketone) composition is fused and melted is varied depending on the composition and the molecular weight of the cyclic poly(phenylene ether ether ketone), the weight fraction of the cyclic poly(phenylene ether ether ketone) included in the cyclic poly(phenylene ether ether ketone) composition and the environment during heating and is thus not unequivocally specifiable. The melting point may, however, be obtained, for example, by analyzing the cyclic poly(phenylene ether ether ketone) composition by a differential scanning calorimeter. The lower limit of the heating temperature is, for example, not lower than 150°C, is preferably not lower than 180°C, is more preferably not lower than 200°C and is furthermore preferably not lower than 220°C. In this temperature range, the cyclic poly(phenylene ether ether ketone) composition is fused and melted, so that the poly(phenylene ether ether ketone) is obtainable in a short time.

The excessively high temperature for the thermal ring-opening polymerization is, on the other hand, likely to cause undesired side reactions, for example, cross-linking reactions between the cyclic poly(phenylene ether ether ketone)s, between the poly(phenylene ether ether ketone)s produced by heating and between the poly(phenylene ether ether ketone) and the cyclic poly(phenylene ether ether ketone), and decomposition reactions. This may result in degrading the properties of the obtained poly(phenylene ether ether ketone). It is accordingly preferable to avoid the temperature that significantly causes such undesired side reactions. The upper limit of the heating temperature is, for example, not higher than 500°C, is preferably not higher than 400°C, is more preferably not higher than 360°C, is furthermore preferably not higher than 335°C, is especially preferably not higher than 300°C. This temperature range is likely to suppress adverse effects of the undesired side reactions on the properties of the obtained poly(phenylene ether ether ketone).

In the case of using the known cyclic poly(phenylene ether ether ketone) described above, i.e., the cyclic poly(phenylene ether ether ketone) having the melting point of higher than 270°C, it takes a long time for the thermal ring-opening polymerization in the above preferable temperature range, due to the high melting point of the cyclic poly(phenylene ether ether ketone). Otherwise the thermal ring-opening polymerization does not proceed, so that it is unlikely to obtain sufficiently highly polymerized poly(phenylene ether ether ketone). The cyclic poly(phenylene ether ether ketone) composition according to the embodiment of the invention that is characterized by the melting point of not higher than 270°C, on the other hand, enables the thermal ring-opening polymerization to efficiently proceed in the above preferable temperature range, so that the sufficiently highly polymerized poly(phenylene ether ether ketone) is obtained. In the production method of the poly(phenylene ether ether ketone) according to the embodiment of the invention, thermal ring-opening polymerization may be performed at the temperature of not higher than the melting point of the obtained poly(phenylene ether ether ketone).

The time of thermal ring-opening polymerization is varied depending on various conditions, for example, the weight fraction and the composition ratio of the cyclic poly(phenylene ether ether ketone) in the used cyclic poly(phenylene ether ether ketone) composition, the heating temperature and the method of thermal ring-opening polymerization and is thus not unequivocally specifiable. It is, however, preferable to set the time of thermal ring-opening polymerization, in order to interfere with the undesired side reactions, such as the cross-linking reactions described above. More specifically, the time of thermal ring-opening polymerization may be, for example, in the range of 0.01 to 100 hours, is preferably in the range of 0.05 to 20 hours and is more preferably in the range of 0.05 to 10 hours. Setting this preferable reaction time is likely to suppress adverse effects of the progress of undesired side reactions such as cross-linking reactions on the properties of the obtained poly(phenylene ether ether ketone).

The production method of poly(phenylene ether ether ketone) by thermal ring-opening polymerization of the

cyclic poly(phenylene ether ether ketone) composition according to the embodiment of the invention may be performed in the absence of or in the presence of a catalyst.

The "catalyst" herein is not specifically limited but may be any compound having the effect of accelerating the thermal ring-opening polymerization reaction of the cyclic poly(phenylene ether ether ketone) composition according to the embodiment of the invention. Any of known catalysts including photopolymerization initiators, radical polymerization initiators, cationic polymerization initiators, anionic polymerization initiators and transition metal catalysts may be used; among them, anionic polymerization initiators are preferable. Examples of the anionic polymerization initiator include inorganic alkali metal salts and organic alkali metal salts. Examples of the inorganic alkali metal salt include alkali metal halides such as sodium fluoride, potassium fluoride, cesium fluoride and lithium chloride. Examples of the organic alkali metal slat include: alkali metal alkoxides such as sodium methoxide, potassium methoxide, sodium ethoxide, potassium ethoxide, sodium tert-butoxide and potassium tert-butoxide; alkali metal phenoxides such as sodium phenoxide, potassium phenoxide, sodium-4-phenoxyphenoxide and potassium-4-phenoxyphenoxide; and alkali metal acetates such as lithium acetate, sodium acetate and potassium acetate.

The anionic polymerization initiator is expected to have the catalytic action through nucleophilic attack to the cyclic poly(phenylene ether ether ketone) composition. Accordingly, compounds having the nucleophilic attack performances equivalent to those of these anionic polymerization initiators may also be used as the catalyst. The compound having such nucleophilic attack performance may be a polymer having an anionic polymerizable end. Any of these anionic polymerization initiators may be used alone, or two or more of these anionic polymerization initiators may be used as a mixture. The thermal ring-opening polymerization of the cyclic poly(phenylene ether ether ketone) composition in the presence of any of these preferable catalysts is likely to obtain poly(phenylene ether ether ketone) in a short time. More specifically, the heating time of the thermal ring-opening polymerization is, for example, not greater than 2 hours, is preferably not greater than 1 hour and is more preferably not greater than 0.5 hours.

The amount of the catalyst used differs according to the molecular weight of the target poly(phenylene ether ether ketone) and the type of the catalyst. The following formula shows the primary structural unit of the cyclic poly(phenylene ether ether ketone).

[Chem. 8]

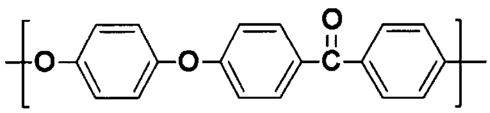

The amount of the catalyst is generally 0.001 to 20 mol%, is preferably 0.005 to 15 mol% and is more preferably 0.01 to 10 mol%, relative to 1 mol of the repeating unit in the primary structural unit of the cyclic poly(phenylene ether ether ketone). Addition of the amount of the catalyst in this preferable range is likely to cause the thermal ring-opening polymerization of the cyclic poly(phenylene ether ether ketone) composition to proceed in a short time.

The procedure of adding the catalyst may be simple addition, but it is preferable to add the catalyst to the cyclic poly(phenylene ether ether ketone) composition and then homogeneously disperse the added catalyst. For example, a method of mechanical dispersion or a dispersion method using a solvent may be employed as the method of homogeneous dispersion. The method of mechanical dispersion may be specifically a method using a pulverizer, a stirrer, a mixer, a shaker or a mortar. The dispersion method using the solvent may be specifically a method that dissolves or disperses the cyclic poly(phenylene ether ether ketone) composition in an adequate solvent, adds the catalyst and subsequently removes the solvent. With respect to dispersion of the catalyst, in the case of a solid catalyst, the average particle diameter of the polymerization catalyst is preferably not greater than 1 mm, in order to enable more homogeneous dispersion of the catalyst. The "average particle diameter" herein means an average value of measured particle diameters with respect to any 100 particles in magnified observation with a microscope.

The thermal ring-opening polymerization of the cyclic poly(phenylene ether ether ketone) composition may be performed in a solvent or under a condition substantially free from a solvent. It is, however, preferable to perform the thermal ring-opening polymerization under the condition substantially free from the solvent, since this is likely to enable a temperature rise in a short time, a high reaction rate and production of poly(phenylene ether ether ketone) in a short time. The "condition substantially free from the solvent" herein indicates that the content of the solvent in the cyclic poly(phenylene ether ether ketone) composition is not higher than 20% by weight, is preferably not higher than 10% by weight and is more preferably not higher than 5% by weight.

The heating method is not specifically limited but may be any method that uses any apparatus having a heating mechanism; for example, a method using a mold used for production of a molded product or a method using an extruder or a melt kneading machine, as well as a method using a conventional polymerization reactor. Any known technique, for example, a batch system or a continuous system may be employed for heating.

The atmosphere in the thermal ring-opening polymerization of the cyclic poly(phenylene ether ether ketone) composition is preferably a non-oxidizing atmosphere. It is also preferable to perform the thermal ring-opening polymerization under a reduced pressure condition. When the thermal ring-opening polymerization is performed under the reduced pressure condition, it is preferable to achieve the reduced pressure condition after once controlling the atmosphere in the reaction system to the non-oxidizing atmosphere. This is likely to suppress the occurrence of undesired side reactions, for example, cross-linking reactions between the cyclic poly(phenylene ether ether ketone)s, between the poly(phenylene ether ether ketone)s produced by the thermal ring-opening polymerization and between the poly(phenylene ether ether ketone) and the cyclic poly(phenylene ether ether ketone), and decomposition reactions.

The "non-oxidizing atmosphere" herein indicates an atmosphere that has the oxygen concentration of not higher than 5% by volume, preferably has the oxygen concentration of not higher than 2% by volume or more preferably does not substantially contain oxygen, in a gas phase, to which the cyclic poly(phenylene ether ether ketone) is exposed. More specifically, the non-oxidizing atmosphere is an inert gas atmosphere such as nitrogen, helium or argon. Among them, nitrogen atmosphere is especially preferable in terms of the economical efficiency and the easiness of handling.

The "reduced pressure condition" herein indicates that the pressure in the reaction system is lower than the atmospheric pressure. The upper limit of the pressure is preferably not greater than 50 kPa, is more preferably not greater than 20 kPa and is furthermore preferably not greater than 10 kPa. The lower limit may be not less than 0.1 kPa and is more preferably not less than 0.2 kPa. Under the reduced pressure condition of the desired lower limit or above, cyclic compounds of low molecular weights included in the cyclic poly(phenylene ether ether ketone) composition are unlikely to be vaporized. Under the reduced pressure condition of the desired upper limit or below, on the other hand, the undesired side reactions such as cross-linking reactions are unlikely to occur.

Heating of the cyclic poly(phenylene ether ether ketone) composition described above may be performed in the coexistence of a fibrous material. The fibrous material herein indicates a fine threadlike material and is preferably any material having an elongated structure like natural fibers. A composite material structure including poly(phenylene ether ether ketone) and the fibrous material can be readily produced by converting the cyclic poly(phenylene ether ether ketone) composition into the poly(phenylene ether ether ketone) in the presence of the fibrous material. This structure is reinforced by the fibrous material and is thus likely to have, for example, better mechanical properties, compared with the poly(phenylene ether ether ketone) alone.

Among fibrous materials, it is preferable to use long-staple reinforcing fibers. This enables production of highly-reinforced poly(phenylene ether ether ketone). In general, in the case of production of a composite material structure including a resin and a fibrous material, the high melt viscosity of the resin often causes the poor wettability of the resin with the fibrous material and thereby fails to produce a homogeneous composite material or to achieve expected mechanical properties.

The "wettability" herein means that a fluid material such as a melt resin and a solid substrate such as a fibrous compound maintain a good physical contact in order to prevent the air or another gas from being substantially trapped between the fluid material and the solid substrate. Here the lower viscosity of the fluid material results in the better wettability with the solid substrate.

The cyclic poly(phenylene ether ether ketone) composition according to the embodiment of the invention has the remarkably lower melt viscosity than those of general thermoplastic resins, for example, poly(phenylene ether ether ketone) and is thereby likely to have the good wettability with the fibrous material. The production method of poly(phenylene ether ether ketone) according to the embodiment of the invention converts the cyclic poly(phenylene ether ether ketone) composition into poly(phenylene ether ether ketone), after achieving the good wettability of the cyclic poly(phenylene ether ether ketone) composition with the fibrous material. This readily produces a complex material structure having the good wettability of the poly(phenylene ether ether ketone) with the fibrous material.

The fibrous material is preferably the long-staple reinforcing fibers as described above. The reinforcing fibers used according to the embodiment of the invention are not specifically limited, but preferably used reinforcing fibers are fibers of good heat resistance and good tensile strength generally used as high-performance reinforcing fibers. Examples of such reinforcing fibers include glass fibers, carbon fibers, graphite fibers, aramid fibers, silicon carbide fibers, alumina fibers and boron fibers. Among them, most preferable are carbon fibers and graphite fibers having good specific strength and good specific modulus and significant contribution to weight reduction. As the carbon fibers and the graphite fibers, any types of carbon fibers and graphite fibers may be used according to their applications, but most suitable are carbon fibers of high strength and high ductility having the tensile strength of not lower than 450 $Kgf/mm^2$ and the tensile ductility of not lower than 1.6%.

When the long-staple reinforcing fibers are used, their length is preferably not shorter than 5 cm. The length of this range readily enables the strength of the reinforcing fibers to be sufficiently provided to the composite material. The carbon fibers or graphite fibers may be used in the form of mixtures with other reinforcing fibers. The shape and the array of the reinforcing fibers are not limited, but the reinforcing fibers may be, for example, unidirectional fibers, random-directional fibers, sheet-like fibers, mat-like fibers, fabric-like fibers or braid-like fibers. The reinforcing fibers having the unidirectionally adjusted array are most suitable for applications that need the specifically high specific strength

and specific modulus. The easily-handled cloth-like (fabric-like) array is also suitable for the embodiment of the invention.

The above conversion of the cyclic poly(phenylene ether ether ketone) composition into poly(phenylene ether ether ketone) may be performed in the presence of a filler. The filler may be, for example, non-fibrous glass, non-fibrous carbon, or an inorganic filler such as calcium carbonate, titanium oxide or alumina.

(11) Applications of Cyclic Poly(Phenylene Ether Ether Ketone) Composition

When being blended with a thermoplastic resin, the cyclic poly(phenylene ether ether ketone) composition according to the embodiment of the invention is likely to significantly reduce the melt viscosity of the thermoplastic resin and have the effect of improving the flowability of the thermoplastic resin. This effect is attributed to little intermolecular entanglement, since the cyclic poly(phenylene ether ether ketone) does not have the end structure unlike conventional linear compounds or linear polymers.

The "thermoplastic resin" herein may be may be any of various melt-moldable resins: for example, polyamide resins, polyester resins, polyacetal resins, polycarbonate resins, polyphenylene ether resins, modified polyphenylene ether resins produced by blending or graft polymerizing polyphenylene ether resins with other resins, polyarylate resins, polysulfone resins, polyphenylene sulfide resins, polyethersulfone resins, polyketone resins, polyether ketone resins, polyether ether ketone resins, polyimide resins, polyamide-imide resins, polyetherimide resins, thermoplastic polyurethane resins, high-density polyethylene resins, low-density polyethylene resins, linear low-density polyethylene resins, polypropylene resins, polymethylpentene resins, cyclic olefin resins, poly(1-butene) resins, poly(1-pentene) resins, polymethylpentene resins, ethylene/ $\alpha$-olefin copolymers, copolymers of (ethylene and/or propylene) and (unsaturated carboxylic acid and/or unsaturated carboxylate), polyolefins obtained by substituting at least part of the carboxyl group with a metal ion in copolymers of (ethylene and/or propylene) and (unsaturated carboxylic acid and/or unsaturated carboxylate), block copolymers of conjugated dienes and vinyl aromatic hydrocarbons, hydrides of block copolymers of conjugated dienes and vinyl aromatic hydrocarbons, polyvinyl chloride resins, polystyrene resins, acrylic resins such as polyacrylate resins and polymethacrylate resins, acrylonitrile copolymers containing acrylonitrile as the major component, acrylonitrile-butadiene-styrene (ABS) resins, acrylonitrile-styrene (AS) resins, cellulose-based resins such as cellulose acetate, vinyl chloride/ ethylene copolymers, vinyl chloride/ vinyl acetate copolymers, ethylene/ vinyl acetate copolymers and saponified ethylene/ vinyl acetate copolymers. Any of these resins may be used alone, or two or more of these resins may be used in combination as a polymer alloy.

The cyclic poly(phenylene ether ether ketone) composition according to the embodiment of the invention may be mixed with any of these thermoplastic resins at any arbitrary ratio. The composition ratio is preferably 70 to 99.9% by weight of the thermoplastic resin and 0.1 to 30% by weight of the cyclic poly(phenylene ether ether ketone) composition, is more preferably 90 to 99.9% by weight of the thermoplastic resin and 0.1 to 10% by weight of the cyclic poly(phenylene ether ether ketone) composition and is furthermore preferably 95 to 99.5% by weight of the thermoplastic resin and 0.5 to 5% by weight of the cyclic poly(phenylene ether ether ketone) composition.

Melt kneading technique is preferably employed for the production method of a thermoplastic resin composition of the cyclic poly(phenylene ether ether ketone) composition according to the embodiment of the invention and the thermoplastic resin. Any known method may be used for melt kneading. For example, the thermoplastic resin and the cyclic poly(phenylene ether ether ketone) composition may be melt kneaded to a resin composition at temperatures of not lower than their melting points by using, for example, a Banbury mixer, a rubber mill, a kneader, a single screw extruder or a twin screw extruder. Especially preferable is a method using a twin screw extruder.

Any of various kneading methods may be employed for kneading: for example, 1) a method of simultaneously kneading the thermoplastic resin and the cyclic poly(phenylene ether ether ketone) composition; and 2) a method of producing a resin composition of the thermoplastic resin including a high concentration of the cyclic poly(phenylene ether ether ketone) composition (master pellets), adding the thermoplastic resin to this resin composition to adjust the concentration of the cyclic poly(phenylene ether ether ketone) composition to a specified concentration and melt kneading the mixture (master pellet method).

The cyclic poly(phenylene ether ether ketone) composition according to the embodiment of the invention is characterized by the low melting point of not lower than 270°C. This enables a low kneading temperature to be set in the melt kneading process for production of the thermoplastic resin composition and is thus likely to facilitate melt kneading with the thermoplastic resin.

The thermoplastic resin composition thus obtained may be molded and processed to and used as various molded products by any arbitrary method, for example, generally known techniques such as injection molding, injection compression molding, compression molding, extrusion molding, blow molding, press molding and spinning. The molded products used include injection molded products, extrusion molded products, blow molded products, films, sheets and fibers. The films used may be various films such as unstretched films, uniaxially stretched films and biaxially stretched films. The fibers used may be various fibers such as undrawn yarns, drawn yarns and ultradrawn yarns.

Examples

The embodiments of the invention are described more specifically below with reference to examples. These examples are, however, only illustrative and not restrictive in any sense.

Various physical properties were measured by high-performance liquid chromatography, a differential scanning calorimeter (DSC), an infrared spectroscopy analyzer (IR) and an Ostwald viscosimeter. The quantitative analysis of cyclic poly(phenylene ether ether ketone) was performed by high-performance liquid chromatography. The detailed conditions of analysis are given below.

(High-performance liquid chromatography)

Apparatus: LC-10Avp series manufactured by SHIMADZU CORPORATION;
Column: Mightysil RP-18GP150-4.6;
Detector: photodiode array detector (using UV= 270 nm);
Column Temperature: 40° C;
Sample Concentration: 0.1% by weight of THF solution; and
Mobile Phase: THF/ 0.1% by weight of trifluoroacetic acid aqueous solution.

(Differential scanning calorimeter)

Apparatus: Robot DSC manufactured by Seiko Instruments Inc.

(Infrared spectroscopy analyzer)

Apparatus: Perkin Elmer System 2000 FT-IR
Sample Preparation: KBr Method

(Viscosimetry)

Viscosimeter: Ostwald viscosimeter (manufactured by Asahi Glassplant Inc.;
Solvent: 98% by weight sulfuric acid;
Sample Concentration: 0.1 g/dL (weight of sample / volume of solvent);
Measurement Temperature: 25°C;
Calculation Formula of Reduced Viscosity: $\eta$= {(t/t0) - 1}/ C;
t: the number of seconds when the sample solution passes through;
t0: the number of seconds when the solvent passes through; and
C: the concentration of the solution

(MALDI-TOF-MS)

Apparatus: AXIMA-TOF2 manufactured by SHIMADZU

CORPORATION

(Mass spectrometry)

Apparatus: M-1200H manufactured by Hitachi, Ltd.

[Reference Example 1]

In an autoclave equipped with an agitator, 1.1 kg (5 mol) of 4,4'-difluorobenzophenone, .0.55 kg (5 mol) of hydroquinone, 0.69 kg (5 mol) of potassium carbonate and 50 L of N-methyl-2-pyrrolidone were mixed. The volume of N-methyl-2-pyrrolidone relative to 1.0 mol of the benzene ring component in the mixture was 3.33 liters.

After the reaction vessel was sealed under nitrogen gas at room temperature and ordinary pressure, the reaction proceeded while the temperature was raised from room temperature to 140°C, was kept at 140°C for 1 hour, was subsequently raised to 180°C, was kept at 180°C for 3 hours, was subsequently raised to 250°C and was kept at 250°C for 5 hours. On completion of the reaction, and subsequent cooling to room temperature and a reaction product was prepared.

About 0.2 g of the obtained reaction product was weighed and diluted with about 4.5 g of THF. A sample of high-performance liquid chromatography was prepared through separation removal of a THF insoluble component by filtration and was subjected to analysis of the reaction product. As a result of the analysis, production of seven different cyclic poly(phenylene ether ether ketone)s having consecutive repeating numbers m= 2 to 8 was confirmed. The yield of the cyclic poly(phenylene ether ether ketone) mixture relative to hydroquinone calculated by the absolute calibration method was 15.0%.

A 50 kg aliquot of this obtained reaction product was mixed with 150 kg of 1% by weight of an acetic acid aqueous solution and was slurried by stirring. The slurry was then heated to 70°C and was continuously stirred for 30 minutes. A solid substance was obtained by filtering the slurry through a filter. The obtained solid substance (S1) was dispersed in 50 kg of deionized water (S2), was kept at 70°C for 30 minutes and was filtrated (S3) to have a solid substance. The above operations (S1) to (S3) were performed two more times. In other words the above operations (S1) to (S3) were repeated three times. The resulting solid substance was vacuum dried overnight at 70°C, and about 1.3 kg of a dried solid matter (mixture (a-r1)) was obtained.

Subsequently, 1.3 kg of the dried solid matter obtained above was subjected to extraction using 30 kg of chloroform at 80°C for 5 hours. After the extraction, chloroform was distilled out from the obtained extract by solid-liquid separation. The residue was vacuum dried at 70°C for 3 hours, so that 0.2 kg of a white solid matter (mixture (b-r1)) was obtained.

This white solid matter was identified as a compound having phenylene ether ketone units from absorption spectra by infrared spectroscopy. This white solid matter was also identified as a cyclic poly(phenylene ether ether ketone) composition including as its primary component a cyclic poly(phenylene ether ether ketone) mixture of seven different cyclic poly(phenylene ether ether ketone)s having consecutive repeating numbers m= 2 to 8, based on the results of mass spectroscopy (apparatus: M-1200H manufactured by Hitachi, Ltd.) after component separation by high-performance liquid chromatography and molecular weight information by MALDI-TOF-MS. Additionally, according to the results of analyses of the obtained cyclic poly(phenylene ether ether ketone) composition, the weight fraction of the cyclic poly(phenylene ether ether ketone) mixture in the cyclic poly(phenylene ether ether ketone) composition was 88%; the melting point of the cyclic poly(phenylene ether ether ketone) composition was 160°C; and the reduced viscosity of the cyclic poly(phenylene ether ether ketone) composition was less than 0.02 dL/g.

A chloroform-insoluble solid component obtained by the above chloroform extraction and solid-liquid separation was vacuum dried overnight at 70°C, so that 1.1 kg of an off-white solid substance was obtained. As the result of analysis, this solid substance was identified as linear poly(phenylene ether ether ketone) from absorption spectra by infrared spectroscopy. This proves that the mixture prepared by the above method was a mixture including linear poly(phenylene ether ether ketone) and cyclic poly(phenylene ether ether ketone).

[Example 1]

The following describes a recovery method of a cyclic poly(phenylene ether ether ketone) composition from the mixture (a-r1) prepared in Reference Example 1 by using acetone as the ketone-based solvent.

The extraction was performed at the bath temperature of 80°C for 5 hours with respect to 20 g of the mixture (a-r1) obtained by the method described in Reference Example 1 by using 500 g of acetone. After the extraction, an extract solution was recovered through solid-liquid separation by filtration. The resulting extract solution was heated under reduced pressure for removal of acetone and was then vacuum dried at 70°C for 3 hours, so that 3. 1 g of a white solid matter was obtained.

This white solid matter was identified as a compound having phenylene ether ketone units from absorption spectra by infrared spectroscopy. This white solid matter was also identified as a cyclic poly(phenylene ether ether ketone) composition including as its primary component a cyclic poly(phenylene ether ether ketone) mixture of five different cyclic poly(phenylene ether ether ketone)s having consecutive repeating numbers m= 2 to 6, based on the

results of mass spectroscopy (apparatus: M-1200H manufactured by Hitachi, Ltd.) after component separation by high-performance liquid chromatography and molecular weight information by MALDI-TOF-MS. Additionally, according to the results of analyses of the obtained cyclic poly(phenylene ether ether ketone) composition, the weight fraction of the cyclic poly(phenylene ether ether ketone) mixture in the cyclic poly(phenylene ether ether ketone) composition was 90%; the melting point of the cyclic poly(phenylene ether ether ketone) composition was 163°C; and the reduced viscosity of the cyclic poly(phenylene ether ether ketone) composition was less than 0.02 dL/g.

According to comparison with Reference Example 1, it is shown that using acetone as a solvent, to which the mixture (a-r1) is exposed, increases the weight fraction of the cyclic poly(phenylene ether ether ketone) mixture in the obtained cyclic poly(phenylene ether ether ketone) composition.

[Example 2]

The extraction was performed at the bath temperature of 80°C for 5 hours with respect to 20 g of the mixture (a-r1) obtained by the method described in Reference Example 1 by using 500 g of acetone. After the extraction, an extract solution was recovered through solid-liquid separation by filtration. The resulting extract solution was heated under reduced pressure for removal of acetone and was then vacuum dried at 70°C for 3 hours, so that 3. 1 g of a white solid matter was obtained. Operations of dispersing the obtained white solid matter in about 100 g of methanol and recovering a solid substance through solid-liquid separation by filtration were repeated three times. The resulting solid substance was vacuum dried at 70°C for 3 hours, so that 3.0 g of a white solid matter was recovered.

This white solid matter was identified as a compound having phenylene ether ketone units from absorption spectra by infrared spectroscopy. This white solid matter was also identified as a cyclic poly(phenylene ether ether ketone) composition including as its primary component a cyclic poly(phenylene ether ether ketone) mixture of five different cyclic poly(phenylene ether ether ketone)s having consecutive repeating numbers m= 2 to 6, based on the results of mass spectroscopy (apparatus: M-1200H manufactured by Hitachi, Ltd.) after component separation by high-performance liquid chromatography and molecular weight information by MALDI-TOF-MS. Additionally, according to the results of analyses of the obtained cyclic poly(phenylene ether ether ketone) composition, the weight fraction of the cyclic poly(phenylene ether ether ketone) mixture in the cyclic poly(phenylene ether ether ketone) composition was 91%; the melting point of the cyclic poly(phenylene ether ether ketone) composition was 163°C; and the reduced viscosity of the cyclic poly(phenylene ether ether ketone) composition was less than 0.02 dL/g.

It is shown that re-purification of the cyclic poly(phenylene ether ether ketone) composition recovered by acetone extraction by using a solvent (methanol) having low solubility for the cyclic poly(phenylene ether ether ketone) mixture increases the weight fraction of the cyclic poly(phenylene ether ether ketone) mixture in the cyclic poly(phenylene ether ether ketone) composition.

[Example 3]

The extraction was performed at the bath temperature of 80°C for 5 hours with respect to 20 g of the mixture (a-r1) obtained by the method described in Reference Example 1 by using 500 g of acetone. After the extraction, an extract solution was recovered through solid-liquid separation by filtration. The resulting extract solution was heated under reduced pressure for removal of acetone and was then vacuum dried at 70°C for 3 hours, so that 3. 2 g of a white solid matter was obtained. After addition of 30 g of acetone to the obtained white solid matter, a fluid dispersion was prepared by using an ultrasonic bath and was added dropwise to 900 g of methanol. A precipitate component thus obtained was filtrated with filter paper having an average pore size of 1 μm and was then vacuum dried at 70°C for 3 hours, so that 3. 1 g of a white solid matter was obtained.

This white solid matter was identified as a compound having phenylene ether ketone units from absorption spectra by infrared spectroscopy. This white solid matter was also identified as a cyclic poly(phenylene ether ether ketone) composition including as its primary component a cyclic poly(phenylene ether ether ketone) mixture of five different cyclic poly(phenylene ether ether ketone)s having consecutive repeating numbers m= 2 to 6, based on the results of mass spectroscopy (apparatus: M-1200H manufactured by Hitachi, Ltd.) after component separation by high-performance liquid chromatography and molecular weight information by MALDI-TOF-MS. Additionally, according to the results of analyses of the obtained cyclic poly(phenylene ether ether ketone) composition, the weight fraction of the cyclic poly(phenylene ether ether ketone) mixture in the cyclic poly(phenylene ether ether ketone) composition was 94%; the melting point of the cyclic poly(phenylene ether ether ketone) composition was 178°C; and the reduced viscosity of the cyclic poly(phenylene ether ether ketone) composition was less than 0.02 dL/g.

It is shown that re-purification of the cyclic poly(phenylene ether ether ketone) composition recovered by acetone extraction by using a solvent (methanol) having low solubility for the cyclic poly(phenylene ether ether ketone) mixture and a small amount of acetone increases the weight fraction of the cyclic poly(phenylene ether ether ketone) mixture in the cyclic poly(phenylene ether ether ketone) composition.

[Example 4]

By using 30 g of acetone, 10 g of the mixture (b-r1) obtained by the method described in Reference Example 1 was mixed with stirring at room temperature for 1 hour. The resulting mixed solution was dispersed in about 600 g of methanol, and a solid substance was recovered through solid-liquid separation by filtration. The obtained solid substance was vacuum dried at 70°C for 3 hours, so that 9.5 g of a white solid batter was recovered.

This white solid matter was identified as a compound having phenylene ether ketone units from absorption spectra by infrared spectroscopy. This white solid matter was also identified as a cyclic poly(phenylene ether ether ketone) composition including as its primary component a cyclic poly(phenylene ether ether ketone) mixture of seven different cyclic poly(phenylene ether ether ketone)s having consecutive repeating numbers m= 2 to 8, based on the results of mass spectroscopy (apparatus: M-1200H manufactured by Hitachi, Ltd.) after component separation by high-performance liquid chromatography and molecular weight information by MALDI-TOF-MS. Additionally, according to the results of analyses of the obtained cyclic poly(phenylene ether ether ketone) composition, the weight fraction of the cyclic poly(phenylene ether ether ketone) mixture in the cyclic poly(phenylene ether ether ketone) composition was 93%; the melting point of the cyclic poly(phenylene ether ether ketone) composition was 159°C; and the reduced viscosity of the cyclic poly(phenylene ether ether ketone) composition was less than 0.02 dL/g.

It is shown that purification operation of exposing the cyclic poly(phenylene ether ether ketone) composition recovered by chloroform extraction to acetone and subsequently performing solid-liquid separation increases the weight fraction of the cyclic poly(phenylene ether ether ketone) mixture in the cyclic poly(phenylene ether ether ketone) composition.

[Reference Example 2]

The following describes preparation of a mixture including at least cyclic poly(phenylene ether ether ketone) and linear poly(phenylene ether ether ketone) by referring to the method described in Examples of JP 2007-302895A.

In a four-necked flask equipped with an agitator, a nitrogen blowing tube, a Dean-Start apparatus, a condenser tube and a thermometer, a mixture was prepared by mixing 26.2 g (120.1 mmol) of 4,4'-difluorobenzophenone, 13.2 g (119.9 mmol) of hydroquinone, 13. 2 g (124.5 mmol) of sodium carbonate, 0.64 g (4.6 mmol) of potassium carbonate and 69.2 g of diphenylsulfone. The volume of the organic polar solvent to 1.0 mol of the benzene ring component in the mixture was about 0.2 liters.

The reaction proceeded while the mixture was heated to the inner temperature of 320°C and was kept at 320°C for 8 hours. After completion of the reaction and subsequent cooling, a reaction mixture was obtained.

As the result of analysis of the obtained reaction mixture by high-performance liquid chromatography, the yield of the cyclic poly(phenylene ether ether ketone) mixture relative to hydroquinone was 1.9%.

The reaction mixture was washed with ethanol and water for removal of byproduct salts and diphenylsulfone and was subsequently dried in a hot air dryer at 120°C, so that a mixture (a-r2) was obtained.

About 1.0 g of the obtained mixture (a-r2) was subjected to Soxhlet extraction by using 100 g of chloroform at the bath temperature of 80°C for 5 hours. After that, chloroform was distilled out from the obtained extract by solid-liquid separation. The residue was vacuum dried at 70°C for 3 hours, so that 0.02 g of a white solid matter (mixture (b-r2)) was obtained.

As the results of analyses by the same method as that of Reference Example 1, this white solid matter was identified as a cycling poly(phenylene ether ether ketone) composition including seven different cyclic poly(phenylene ether ether ketone)s having consecutive repeating numbers m= 2 to 8. The weight fraction of the cyclic poly(phenylene ether ether ketone) mixture in the cyclic poly(phenylene ether ether ketone) composition was 90%.

A chloroform-insoluble solid component obtained by the above chloroform extraction was vacuum dried overnight at 70°C, so that about 0.97 g of an off-white solid component was obtained. As the result of analysis, this solid component was identified as linear poly(phenylene ether ether ketone) from absorption spectra by infrared spectroscopy. This proves that the mixture (a-r2) prepared by the above method was a mixture including linear poly(phenylene ether ether ketone) and cyclic poly(phenylene ether ether ketone).

[Example 5]

The following describes a recovery method of a cyclic poly(phenylene ether ether ketone) composition from the mixture (a-r2) prepared in Reference Example 2 by using acetone.

By using 100 g of acetone, about 1.0 g of the mixture (a-r2) obtained by the method described in Reference Example 2 was subjected to Soxhlet extraction at the bath temperature of 80°C for 5 hours (thermal filtering operation with thimble). After that, the resulting extract was heated under reduced pressure for removal of acetone and was then vacuum dried at 70°C for 3 hours, so that 0.02 g of a white solid matter was obtained.

As the results of analyses by the same method as that of Reference Example 1, this white solid matter was identified as a cyclic poly(phenylene ether ether ketone) composition including four different cyclic poly(phenylene ether ether ketone)s having consecutive repeating numbers m= 2 to 5. The weight fraction of the cyclic poly(phenylene ether ether ketone) mixture in the cyclic poly(phenylene ether ether ketone) composition was 92%. The melting point of the cyclic poly(phenylene ether ether ketone) composition was 172°C; and the reduced viscosity of the cyclic poly(phenylene ether ether ketone) composition was less than 0.02 dL/g.

[Example 6]

The following describes a recovery method of a cyclic poly(phenylene ether ether ketone) composition from the mixture (a-r2) prepared in Reference Example 2 by using acetone.

By using 200 g of acetone, 20 g of the mixture (a-r2) obtained by the method described in Reference Example 2 was subjected to Soxhlet extraction at the bath temperature of 80°C for 5 hours (thermal filtering operation with extraction thimble). After that, the resulting extract was heated under reduced pressure for removal of acetone and was then vacuum dried at 70°C for 3 hours, so that 0.4 g of a white solid matter was obtained.

After addition of 4 g of acetone to the obtained white solid matter, a fluid dispersion was prepared by using an ultrasonic bath and was added dropwise to 120 g of methanol. A precipitate component thus obtained was filtrated with filter paper having an average pore size of 1 μm and was then vacuum dried at 70°C for 3 hours, so that 0.4 g of a white solid matter was obtained.

As the results of analyses by the same method as that of Reference Example 1, this white solid matter was identified as a cyclic poly(phenylene ether ether ketone) composition including four different cyclic poly(phenylene ether ether ketone)s having consecutive repeating numbers m= 2 to 5. The weight fraction of the cyclic poly(phenylene ether ether ketone) mixture in the cyclic poly(phenylene ether ether ketone) composition was 94%. The melting point of the cyclic poly(phenylene ether ether ketone) composition was 171°C; and the reduced viscosity of the cyclic poly(phenylene ether ether ketone) composition was less than 0.02 dL/g.

[Example 7]

The following describes a recovery method of a cyclic poly(phenylene ether ether ketone) composition from the mixture (b-r2) prepared by the method described in Reference Example 2.

By using 2 g of acetone, 0.5 g of the mixture (b-r2) prepared by the method described in Reference Example 2 was mixed with stirring at room temperature for 1 hour. The resulting mixed solution was dispersed in about 40 g of methanol, and a solid substance was recovered through solid-liquid separation by filtration. The obtained solid substance was vacuum dried at 70°C for 3 hours, so that 0.5 g of a white solid matter was recovered.

As the results of analyses by the same method as that of Reference Example 1, this white solid matter was identified as a cyclic poly(phenylene ether ether ketone) composition including four different cyclic poly(phenylene ether ether ketone)s having consecutive repeating numbers m= 2 to 5. The weight fraction of the cyclic poly(phenylene ether ether ketone) mixture in the cyclic poly(phenylene ether ether ketone) composition was 94%. The melting point of the cyclic poly(phenylene ether ether ketone) composition was 171°C; and the reduced viscosity of the cyclic poly(phenylene ether ether ketone) composition was less than 0.02 dL/g.

[Reference Example 3]

The following describes preparation by referring to the method described in Examples of Japanese translation of International Patent Application 2007-506833A.

In a four-necked flask equipped with an agitator, a nitrogen blowing tube, a Dean-Start apparatus, a condenser tube and a thermometer, 18.0 g (82.4 mmol) of 4,4'-difluorobenzophenone, 8.8 g (80 mmol) of hydroquinone and 49 g of diphenylsulfone were mixed. The mixture was heated to 140°C with nitrogen flow, so that a substantially colorless solution was obtained. A mixture was prepared by adding 8.5 g (80 mmol) of sodium carbonate and 0.2 g (1.6 mmol) of potassium carbonate at this temperature. The volume of the organic polar solvent to 1.0 mol of the benzene ring component in the mixture was about 0.2 liters.

The reaction proceeded while the mixture was heated to the inner temperature of 200°C, was kept at 200°C for 1 hour, was subsequently heated to 250°C, was kept at 250°C for 1 hour, was subsequently heated to 315°C and was kept at 315°C for 3 hours. After completion of the reaction and subsequent cooling, a reaction mixture (a-r3) was obtained.

As the result of analysis of the obtained reaction mixture (a-r3) by high-performance liquid chromatography, the yield of the cyclic poly(phenylene ether ether ketone) mixture relative to hydroquinone was 1.8%.

The reaction mixture was washed with water and acetone for removal of byproduct salts and diphenylsulfone

and was subsequently dried in a hot air dryer at 120°C, so that a mixture was obtained.

About 1.0 g of the obtained mixture was subjected to Soxhlet extraction by using 100 g of chloroform at the bath temperature of 80°C for 5 hours. After that, chloroform was distilled out from the obtained extract by solid-liquid separation. The residue was vacuum dried at 70°C for 3 hours, so that 0.02 g of a white solid matter (mixture (b-r3)) was obtained.

As the results of analyses by the same method as that of Reference Example 1, this white solid matter was identified as a cycling poly(phenylene ether ether ketone) composition including seven different cyclic poly(phenylene ether ether ketone)s having consecutive repeating numbers m= 2 to 8. The weight fraction of the cyclic poly(phenylene ether ether ketone) mixture was 87%.

A chloroform-insoluble solid component obtained by the above chloroform extraction was vacuum dried overnight at 70°C, so that about 0.97 g of an off-white solid component was obtained. As the result of analysis, this solid component was identified as linear poly(phenylene ether ether ketone) from absorption spectra by infrared spectroscopy. This proves that the mixture (a-r3) prepared by the above method was a mixture including linear poly(phenylene ether ether ketone) and cyclic poly(phenylene ether ether ketone).

[Example 8]

The following describes a recovery method of a cyclic poly(phenylene ether ether ketone) composition from the mixture (a-r3) prepared in Reference Example 3 by using acetone.

By using 100 g of acetone, about 4.5 g of the mixture (a-r3) obtained by the method described in Reference Example 3 was subjected to Soxhlet extraction at the bath temperature of 80°C for 5 hours (thermal filtering operation with extraction thimble). After that, the resulting extract was heated under reduced pressure for removal of acetone and was then vacuum dried at 70°C for 3 hours, so that 2.62 g of a solid substance was obtained. Operations of dispersing the obtained solid substance in about 100 g of ethanol and recovering a solid substance through solid-liquid separation by filtration were repeated three times. The resulting solid substance was vacuum dried at 70°C for 3 hours, so that 0.02 g of a white solid matter was recovered.

As the results of analyses by the same method as that of Reference Example 1, this white solid matter was identified as a cyclic poly(phenylene ether ether ketone) composition including four different cyclic poly(phenylene ether ether ketone)s having consecutive repeating numbers m= 2 to 5. The weight fraction of the cyclic poly(phenylene ether ether ketone) mixture in the cyclic poly(phenylene ether ether ketone) composition was 92%. The melting point of the cyclic poly(phenylene ether ether ketone) composition was 172°C; and the reduced viscosity of the cyclic poly(phenylene ether ether ketone) composition was less than 0.02 dL/g.

[Example 9]

The following describes a recovery method of a cyclic poly(phenylene ether ether ketone) composition from the mixture (a-r3) prepared in Reference Example 3 by using acetone.

By using 1 kg of acetone, 45 g of the mixture (a-r3) obtained by the method described in Reference Example 3 was subjected to Soxhlet extraction at the bath temperature of 80°C for 5 hours (thermal filtering operation with extraction thimble). After that, the resulting extract was heated under reduced pressure for removal of acetone and was then vacuum dried at 70°C for 3 hours, so that 26.2 g of a solid substance was obtained. Operations of dispersing the obtained solid substance in about 1 kg of ethanol and recovering a solid substance through solid-liquid separation by filtration were repeated three times. The resulting solid substance was vacuum dried at 70°C for 3 hours, so that 0.2 g of a white solid matter was recovered.

After addition of 2 g of acetone to the obtained white solid matter, a fluid dispersion was prepared by using an ultrasonic bath and was added dropwise to 60 g of methanol. A precipitate component thus obtained was filtrated with filter paper having an average pore size of 1 μm and was then vacuum dried at 70°C for 3 hours, so that 0.2 g of a white solid matter was obtained.

As the results of analyses by the same method as that of Reference Example 1, this white solid matter was identified as a cyclic poly(phenylene ether ether ketone) composition including four different cyclic poly(phenylene ether ether ketone)s having consecutive repeating numbers m= 2 to 5. The weight fraction of the cyclic poly(phenylene ether ether ketone) mixture in the cyclic poly(phenylene ether ether ketone) composition was 94%. The melting point of the cyclic poly(phenylene ether ether ketone) composition was 168°C; and the reduced viscosity of the cyclic poly(phenylene ether ether ketone) composition was less than 0.02 dL/g.

[Example 10]

The following describes a recovery method of a cyclic poly(phenylene ether ether ketone) composition from

the mixture (a-r3) prepared in Reference Example 3.

By using 1 kg of chloroform, 45 g of the mixture (a-r3) prepared by the method described in Reference Example 3 was subjected to Soxhlet extraction at the bath temperature of 80°C for 5 hours (thermal filtering operation with extraction thimble). After that, the resulting extract was heated under reduced pressure for removal of chloroform and was then vacuum dried at 70°C for 3 hours, so that 26.2 g of a solid substance was obtained. Operations of dispersing the obtained solid substance in about 1 kg of ethanol and recovering a solid substance through solid-liquid separation by filtration were repeated three times. The resulting solid substance was vacuum dried at 70°C for 3 hours, so that 0.22 g of a white solid matter was recovered. After addition of 2 g of acetone to the obtained white solid matter, a fluid dispersion was prepared by using an ultrasonic bath and was added dropwise to 60 g of methanol. A precipitate component thus obtained was filtrated with filter paper having an average pore size of 1 $\mu$m and was then vacuum dried at 70°C for 3 hours, so that 0.22 g of a white solid matter was obtained.

As the results of analyses by the same method as that of Reference Example 1, this white solid matter was identified as a cyclic poly(phenylene ether ether ketone) composition including six different cyclic poly(phenylene ether ether ketone)s having consecutive repeating numbers m= 2 to 7. The weight fraction of the cyclic poly(phenylene ether ether ketone) mixture in the cyclic poly(phenylene ether ether ketone) composition was 93%. The melting point of the cyclic poly(phenylene ether ether ketone) composition was 170°C; and the reduced viscosity of the cyclic poly(phenylene ether ether ketone) composition was less than 0.02 dL/g.

[Reference Example 4]

The following describes preparation of poly(phenylene ether ether ketone) by referring to the method described in Examples of JP 2010-95614A.

In a four-necked flask equipped with an agitator, a nitrogen blowing tube, a Dean-Start apparatus, a condenser tube and a thermometer, 22.2 g (101.5 mmol) of 4,4'-difluorobenzophenone, 11.0 g (100.0 mmol) of hydroquinone and 164 g of sulfolane were mixed. The mixture was heated to 80°C with nitrogen flow, so that a substantially colorless solution was obtained. A mixture was prepared by adding 14.0 g (101.0 mmol) of potassium carbonate at this temperature. The volume of the organic polar solvent to 1.0 mol of the benzene ring component in the mixture was about 0.54 liters.

The reaction proceeded while the mixture was heated to the inner temperature of 265°C and was kept at 265°C for 4 hours. After completion of the reaction and subsequent cooling, a reaction mixture was obtained.

As the result of analysis of the obtained reaction mixture by high-performance liquid chromatography, the yield of the cyclic poly(phenylene ether ether ketone) mixture relative to hydroquinone was 5.9%.

The reaction mixture was washed with water for removal of byproduct salts and sulfolane and was subsequently dried in a hot air dryer at 120°C, so that a mixture (a-r4) was obtained.

About 1.0 g of the obtained mixture (a-r4) was subjected to Soxhlet extraction by using 100 g of chloroform at the bath temperature of 80°C for 5 hours. After that, chloroform was distilled out from the obtained extract by solid-liquid separation. The residue was vacuum dried at 70°C for 3 hours, so that 0.06 g of a white solid matter (mixture (b-r4)) was obtained.

As the results of analyses by the same method as that of Reference Example 1, this white solid matter was identified as a cyclic poly(phenylene ether ether ketone) composition including seven different cyclic poly(phenylene ether ether ketone)s having consecutive repeating numbers m= 2 to 8. The weight fraction of the cyclic poly(phenylene ether ether ketone) mixture in the cyclic poly(phenylene ether ether ketone) composition was 89%.

A chloroform-insoluble solid component obtained by the above chloroform extraction was vacuum dried overnight at 70°C, so that about 0.93 g of an off-white solid component was obtained. As the result of analysis, this solid component was identified as linear poly(phenylene ether ether ketone) from absorption spectra by infrared spectroscopy. This proves that the mixture (a-r4) prepared by the above method was a mixture including linear poly(phenylene ether ether ketone) and cyclic poly(phenylene ether ether ketone).

[Example 11]

The following describes a recovery method of a cyclic poly(phenylene ether ether ketone) composition from the mixture (a-r4) prepared in Reference Example 4 by using acetone.

By using 100 g of acetone, about 1.0 g of the mixture (a-r4) obtained by the method described in Reference Example 4 was subjected to Soxhlet extraction at the bath temperature of 80°C for 5 hours (thermal filtering operation with extraction thimble). After that, the resulting extract was heated under reduced pressure for removal of acetone and was then vacuum dried at 70°C for 3 hours, so that 0.05 g of a white solid matter was obtained.

As the results of analyses by the same method as that of Reference Example 1, this white solid matter was identified as a cyclic poly(phenylene ether ether ketone) composition including four different cyclic poly(phenylene ether ether ketone)s having consecutive repeating numbers m= 2 to 5. The weight fraction of the cyclic poly(phenylene ether

ether ketone) mixture in the cyclic poly(phenylene ether ether ketone) composition was 92%. The melting point of the cyclic poly(phenylene ether ether ketone) composition was 173°C; and the reduced viscosity of the cyclic poly(phenylene ether ether ketone) composition was less than 0.02 dL/g.

[Example 12]

The following describes a recovery method of a cyclic poly(phenylene ether ether ketone) composition from the mixture (a-r4) prepared in Reference Example 4 by using acetone.

By using 100 g of acetone, about 10 g of the mixture (a-r4) obtained by the method described in Reference Example 4 was subjected to Soxhlet extraction at the bath temperature of 80°C for 5 hours (thermal filtering operation with extraction thimble). After that, the resulting extract was heated under reduced pressure for removal of acetone and was then vacuum dried at 70°C for 3 hours, so that 0.5 g of a white solid matter was obtained.

After addition of 5 g of acetone to the obtained white solid matter, a fluid dispersion was prepared by using an ultrasonic bath and was added dropwise to 100 g of methanol. A precipitate component thus obtained was filtrated with filter paper having an average pore size of 1 $\mu$m and was then vacuum dried at 70°C for 3 hours, so that 0.5 g of a white solid matter was obtained.

As the results of analyses by the same method as that of Reference Example 1, this white solid matter was identified as a cyclic poly(phenylene ether ether ketone) composition including four different cyclic poly(phenylene ether ether ketone)s having consecutive repeating numbers m= 2 to 5. The weight fraction of the cyclic poly(phenylene ether ether ketone) mixture in the cyclic poly(phenylene ether ether ketone) composition was 94%. The melting point of the cyclic poly(phenylene ether ether ketone) composition was 168°C; and the reduced viscosity of the cyclic poly(phenylene ether ether ketone) composition was less than 0.02 dL/g.

[Example 13]

The following describes a recovery method of a cyclic poly(phenylene ether ether ketone) composition from the mixture (b-r4) prepared by the method described in Reference Example 4.

By using 2 g of acetone, 0.5 g of the mixture (b-r4) prepared by the method described in Reference Example 4 was mixed with stirring at room temperature for 1 hour. The resulting mixed solution was dispersed in about 40 g of methanol, and a solid substance was recovered through solid-liquid separation by filtration. The obtained solid substance was vacuum dried at 70°C for 3 hours, so that 0.5 g of a white solid batter was recovered.

As the results of analyses by the same method as that of Reference Example 1, this white solid matter was identified as a cyclic poly(phenylene ether ether ketone) composition including seven different cyclic poly(phenylene ether ether ketone)s having consecutive repeating numbers m= 2 to 8. The weight fraction of the cyclic poly(phenylene ether ether ketone) mixture in the cyclic poly(phenylene ether ether ketone) composition was 93%. The melting point of the cyclic poly(phenylene ether ether ketone) composition was 170°C; and the reduced viscosity of the cyclic poly(phenylene ether ether ketone) composition was less than 0.02 dL/g.

[Example 14]

The following describes ring-opening polymerization of a cyclic poly(phenylene ether ether ketone) composition obtained by the recovery method according to the embodiment of the invention.

After 100 mg of powder prepared by mixing the cyclic poly(phenylene ether ether ketone) composition obtained by the method described in Example 1 with 5 mol% of cesium fluoride relative to the repeating unit expressed by a formula -(O-Ph-O-Ph-CO-Ph)- as the primary structural unit of the cyclic poly(phenylene ether ether ketone) was placed in a glass vial, the inside of the vial was replaced with nitrogen. The vial was placed in an electric oven adjusted to the temperature of 350°C and was heated for 60 minutes. After that, the vial was taken out of the oven and was cooled down to room temperature, so that a black solid matter was obtained.

As the result of analysis of the black solid matter using a differential scanning calorimeter, the black solid matter had the melting point of 332°C and the crystallization temperature of 240°C as the thermal properties. Measurement of the reduced viscosity of the black solid matte showed $\eta$=0.5 dL/g.

[Example 15]

The following describes ring-opening polymerization of a cyclic poly(phenylene ether ether ketone) composition obtained by the recovery method according to the embodiment of the invention.

After 100 mg of powder prepared by mixing the cyclic poly(phenylene ether ether ketone) composition obtained by the method described in Example 1 with 5 mol% of cesium fluoride relative to the repeating unit expressed by a

formula -(O-Ph-O-Ph-CO-Ph)- as the primary structural unit of the cyclic poly(phenylene ether ether ketone) was placed in a glass vial, the inside of the vial was replaced with nitrogen. The vial was placed in an electric oven adjusted to the temperature of 320°C and was heated for 60 minutes. After that, the vial was taken out of the oven and was cooled down to room temperature, so that a black solid matter was obtained.

As the result of analysis of the black solid matter using a differential scanning calorimeter, the black solid matter had the melting point of 347°C and the crystallization temperature of 246°C as the thermal properties. Measurement of the reduced viscosity of the black solid matte showed $\eta$=0.5 dL/g.

## Claims

1. A recovery method of a cyclic poly(phenylene ether ether ketone) composition, comprising:

    exposing a mixture including linear poly(phenylene ether ether ketone) and cyclic poly(phenylene ether ether ketone) to a ketone-based solvent and performing solid-liquid separation, so as to recover a cyclic poly(phenylene ether ether ketone) composition including a cyclic poly(phenylene ether ether ketone) mixture of cyclic poly(phenylene ether ether ketone)s which are expressed by General Formula (I) and have different repeating numbers m:

    [Chem. 1]

    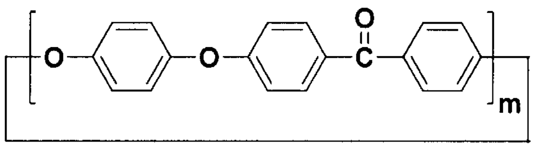

    (I)

    (wherein m in (I) is an integral number of 2 to 40.)

2. The recovery method of the cyclic poly(phenylene ether ether ketone) composition according to claim 1, wherein the cyclic poly(phenylene ether ether ketone) mixture is a mixture of cyclic poly(phenylene ether ether ketone)s having three or more different integral numbers as the different repeating numbers m.

3. The recovery method of the cyclic poly(phenylene ether ether ketone) composition according to either one of claims 1 and 2, wherein
the cyclic poly(phenylene ether ether ketone) mixture is a mixture of cyclic poly(phenylene ether ether ketone)s having three or more consecutive different integral numbers as the different repeating numbers m.

4. The recovery method of the cyclic poly(phenylene ether ether ketone) composition according to any one of claims 1 to 3, wherein
the cyclic poly(phenylene ether ether ketone) composition includes 60% by weight or more of the cyclic poly(phenylene ether ether ketone)s.

5. The recovery method of the cyclic poly(phenylene ether ether ketone) composition according to any one of claims 1 to 4, wherein
the cyclic poly(phenylene ether ether ketone) composition has a melting point of 270°C or lower.

6. The recovery method of the cyclic poly(phenylene ether ether ketone) composition according to any one of claims 1 to 5, wherein
the mixture including the linear poly(phenylene ether ether ketone) and the cyclic poly(phenylene ether ether ketone) is a mixture (a) prepared by reaction under heating of a mixture (x) including a dihalogenated aromatic ketone compound, a dihydroxy aromatic compound, a base and an organic polar solvent.

7. The recovery method of the cyclic poly(phenylene ether ether ketone) composition according to claim 6,
the recovery method exposing the mixture (a) to the ketone-based solvent and performing solid-liquid separation, so as to recover the cyclic poly(phenylene ether ether ketone) composition from an obtained filtrate.

8. The recovery method of the cyclic poly(phenylene ether ether ketone) composition according to any one of claims

1 to 5, wherein
the mixture including the linear poly(phenylene ether ether ketone) and the cyclic poly(phenylene ether ether ketone) is a mixture (b) obtained from an organic solvent (A)-soluble component by preparing a mixture (a) by reaction under heating of a mixture (x) including a dihalogenated aromatic ketone compound, a dihydroxy aromatic compound, a base and an organic polar solvent and exposing the prepared mixture (a) to an organic solvent (A).

**9.** The recovery method of the cyclic poly(phenylene ether ether ketone) composition according to claim 8,
the recovery method exposing the mixture (b) to the ketone-based solvent, subsequently exposing the mixture (b) to a solvent having low solubility for a poly(phenylene ether ether ketone) component and miscibility with the ketone-based solvent and subsequently performing solid-liquid separation, so as to recover the cyclic poly(phenylene ether ether ketone) composition.

**10.** The recovery method of the cyclic poly(phenylene ether ether ketone) composition according to either one of claims 8 and 9, wherein
the organic solvent (A) is a solvent having high solubility for the cyclic poly(phenylene ether ether ketone) and low solubility for the linear poly(phenylene ether ether ketone).

**11.** The recovery method of the cyclic poly(phenylene ether ether ketone) composition according to any one of claims 1 to 10, wherein
the ketone-based solvent is acetone and/or methyl ethyl ketone.

**12.** The recovery method of the cyclic poly(phenylene ether ether ketone) composition according to any one of claims 1 to 11, wherein
the ketone-based solvent is acetone.

**13.** A production method of poly(phenylene ether ether ketone) by thermal ring-opening polymerization of the cyclic poly(phenylene ether ether ketone) composition obtained by the recovery method according to any one of claims 1 to 12.

## INTERNATIONAL SEARCH REPORT

International application No.
PCT/JP2012/005896

A. CLASSIFICATION OF SUBJECT MATTER
*C07D323/00*(2006.01)i, *C08G65/40*(2006.01)i

According to International Patent Classification (IPC) or to both national classification and IPC

B. FIELDS SEARCHED

Minimum documentation searched (classification system followed by classification symbols)
C07D323/00, C08G65/40

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
JSTPlus/JMEDPlus/JST7580(JDreamII), CAplus(STN), CASREACT(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | WO 2011/081080 A1 (Toray Industries, Inc.), 07 July 2011 (07.07.2011), example 1 (Family: none) | 1-4,6-13 |
| Y | JONAS, A. et al, PEEK Oligomers: A Model for the Polymer Physical Behavior. 3. Nature of Oligomers in the PEEK Polymer, Macromolecules 1993, Vol.26, p.2674-2678. | 1-4,6-13 |
| Y | CHEN, M. et al, Concise synthesis and characterization of 30-membered macrocyclic monomer for poly(ether ether ketone), Macromolecular Chemistry and Physics, 1996, Vol.197, p.4069-4078. | 1-4,6-13 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 December, 2012 (05.12.12) | 18 December, 2012 (18.12.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2012/005896

C (Continuation). DOCUMENTS CONSIDERED TO BE RELEVANT

| Category* | Citation of document, with indication, where appropriate, of the relevant passages | Relevant to claim No. |
|---|---|---|
| A | COLQUHOUN, H.M. et al, Reversible, Nondegradative Conversion of Crystalline Aromatic Poly(ether ketone)s into Organo-Soluble Poly(ether dithioketal)s, Macromolecules 2009, Vol.42, p.1955-1963. | 1-13 |
| A | CHEN, M. et al, Large-Sized Macrocyclic Monomeric Precursors of Poly(ether ether ketone): Synthesis and Polymerization, Macromolecules 1996, Vol.29, p.5502-5504. | 1-13 |
| A | JP 2-000159 A (IMPERIAL CHEM IND PLC), 05 January 1990 (05.01.1990), entire text & EP 317226 A | 1-13 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP S51119797 A
- JP 2007302895 A
- JP 2010070657 A
- WO 2011081080 A
- JP 2007506833 A
- JP 2010095614 A

### Non-patent literature cited in the description

- *Macromolecules,* 1996, vol. 29, 5502
- *Macromol. Chem. Phys.,* 1996, vol. 197, 4069
- *Macromolecules,* 1993, vol. 26, 2674